# FASCICULE DE BREVET EUROPEEN

(11) **EP 1 743 034 B1**
(45) Date de publication et mention de la délivrance du brevet: **12.12.2007**
(21) Numéro de dépôt: 05769759.1
(22) Date de dépôt: 04.05.2005
(51) Int. Cl.: C12Q 1/37

(54) **SUBSTRATS PEPTIDIQUES RECONNUS PAR LA TOXINE BOTULIQUE DE TYPE A, BONT/A ET LEURS UTILISATIONS**
MITTELS BOTULINUMTOXIN A (BONT/A) ERKENNBARE PEPTIDSUBSTRATE UND VERWENDUNG
PEPTIDE SUBSTRATES RECOGNISABLE BY A BOTULINUM TOXIN A, BONT/A AND THE USE THEREOF

(30) Priorité: 05.05.2004 FR 0404817; 11.02.2005 FR 0501435
(43) Date de publication de la demande: 17.01.2007
(73) Titulaire: Pharmaleads, 75014 Paris (FR)
(72) Inventeur: FOURNIE-ZALUSKI, Marie-Claude, F-75011 Paris (FR); ROQUES, Bernard, Pierre, F-75015 Paris (FR)
(74) Mandataire: Grosset-Fournier, Chantal Catherine
(86) Numéro de dépôt international: PCT/FR2005/001121
(87) Numéro de publication internationale: WO 2005/121354

(56) Documents cités:
- FR-A- 2 809 733
- ANNE C ET AL: "HIGH-THROUGHPUT FLUOROGENIC ASSAY FOR DETERMINATION OF BOTULINUM TYPE B NEUROTOXIN PROTEASE ACTIVITY" ANALYTICAL BIOCHEMISTRY, ACADEMIC PRESS, NEW YORK, NY, US, vol. 291, no. 2, 15 avril 2001 (2001-04-15), pages 253-261, XP001023970 ISSN: 0003-2697
- SOLEILHAC ET AL: "A sensitive and rapid fluorescence-based assay for determination of tetanus toxin peptidase activity" ANALYTICAL BIOCHEMISTRY, ACADEMIC PRESS, SAN DIEGO, CA, US, vol. 241, 1996, pages 120-127, XP002160418 ISSN: 0003-2697
- LUCIANI N ET AL: "HIGHLY SENSITIVE AND SELECTIVE FLUORESCENCE ASSAYS FOR RAPID SCREENING OF ENDOTHELIN-CONVERTING ENZYME INHIBITORS" BIOCHEMICAL JOURNAL, THE BIOCHEMICAL SOCIETY, LONDON, GB, vol. 356, no. 3, 15 juin 2001 (2001-06-15), pages 813-819, XP001025608 ISSN: 0264-6021

### LISTE DE SEQUENCES

<110> PHARMALEADS
<120> SUBSTRAT PEPTIDIQUE RECONNU PAR LA TOXINE BOTULIQUE DE TYPE A, BONT/A ET SON UTILISATION POUR DETECTER ET/OU DOSER LADITE TOXINE OU DES INHIBITEURS DE SON ACTIVITE
<130> BOTA
<160> 27
<170> PatentIn version 3.1
<210> 1
   <211> 206
   <212> PRT
   <213> Homo sapiens
<400> 1
<210> 2
   <211> 17
   <212> PRT
   <213> Séquence artificielle
<220>
   <223> Peptide dérivé de SNAP25
<400> 2
   Ser Asn Lys Thr Arg Ile Asp Glu Ala Asn Gln Arg Ala Thr Lys Met
   1               5                   10                  15
   Leu
<210> 3
   <211> 4
   <212> PRT
   <213> Séquence artificielle
<220>
   <223> Peptide dérivé de SNAP25
<220>
   <221> MISC_FEATURE
   <222> (1) .. (1)
   <223> L-(paranitro)phénylalanine (Nop)
<220>
   <221> MISC_FEATURE
   <222> (4) .. (4)
   <223> L-prénylalanine (Pya)
<400> 3
   Xaa Arg Ala Xaa
   1
<210> 4
   <211> 5
   <212> PRT
   <213> Séquence artificielle
<220>
   <223> Peptide dérivé de SNAP25
<220>
   <221> MISC_FEATURE
   <222> (1)..(1)
   <223> L-(paranitro)phénylalanine (Nop)
<220>
   <221> MISC_FEATLTRE
   <222> (5)..(5)
   <223> L-prénylalanine (Pya)
<400> 4
   Xaa Arg Ala Thr Xaa
   1 5
<210> 5
   <211> 6
   <212> PRT
   <213> Séquence artificielle
<220>
   <223> Peptide dérivé de SNAP25
<220>
   <221> MTSC_FEATURE
   <222> (1)..(1)
   <223> L-(paranitro)phénylalanine (Nop)
<220>
   <221> MISC_FEATURE
   <222> (6) .. (6)
   <223> L-prénylalanine (Pya)
<400> 5
   Xaa Arg Ala Thr Lys Xaa
   1 5
<210> 6
   <211> 21
   <212> PRT
   <213> Séquence artificielle
<220>
   <223> Peptide dérivé de SNAP25
<220>
   <221> MISC_FEATURE
   <222> (1) .. (1)
   <223> Un acide aminé acétylé ou un enchaînement de 2 à 52 acides aminés acétylé du côté N-terminal ou aucun acide aminé
<220>
   <221> MISC_FEATURE
   <222> (2) .. (2)
   <223> Ser ou Ser acétylée si aucun acide aminé du côté N-terminal
<220>
   <221> MISC_FEATURE
   <222> (9)..(9)
   <223> Glu ou Gln
<220>
   <221> MISC_FEATURE
   <222> (12)..(12)
   <223> L-(paranitro)phénylalanine (Nop)
<220>
   <221> MISC_FEATURE
   <222> (15)..(15)
   <223> L-prénylalanine (Pya)
<220>
   <221> MISC_FEATURE
   <222> (17)..(17)
   <223> Nle ou Nle amidifiée si aucun acide aminé en position suivante
<220>
   <221> MISC_FEATURE
   <222> (18)..(18)
   <223> L-leucine ou L-leucine amidifiée si aucun acide aminé en position suivante ou aucun acide aminé
<220>
   <221> MISC_FEATURE
   <222> (19).. (21)
   <223> Gly-Ser-Gly amidifié ou aucun acide aminé
<400> 6
   Xaa Xaa Asn Lys Thr Arg Ile Asp Xaa Ala Asn Xaa Arg Ala Xaa Lys
   1               5                   10                  15
   Xaa Xaa Xaa Xaa Xaa
               20
<210> 7
   <211> 17
   <212> PRT
   <213> Séquence artificielle
<220>
   <223> Séquence dérivée de SNAP25
<220>
   <221> MISC_FEATURE
   <222> (1)..(1)
   <223> L-aspartate acétylée ou L-cystéine acétylée ou aucun acide aminé
<220>
   <221> MISC_FEATURE
   <222> (2) .. (2)
   <223> L-sérine ou L-sérine acétylée si aucun acide aminé en position précédente
<220>
   <221> MISC_FEATURE
   <222> (9) .. (9)
   <223> Glu ou Gln
<220>
   <221> MISC_FEATURE
   <222> (12)..(12)
   <223> L-(paranitro)phénylalanine (Nop)
<220>
   <221> MISC_FEATURE
   <222> (15)..(15)
   <223> L-prénylalanine (Pya)
<220>
   <221> MISC_FEATURE
   <222> (17)..(17)
   <223> Nle ou Nle amidifiée si aucun acide aminé en position 18
<220>
   <221> MISC_FEATURE
   <222> (18)..(18)
   <223> L-leucine amidifiée ou aucun acide aminé
<400> 7
<210> 8
   <211> 17.
   <212> PRT
   <213> Séquence artificielle
<220>
   <223> Peptide dérivé de SNAP25
<220>
   <221> MISC_FEATURE
   <222> (1)..(1)
   <223> L-sérine acétylée
<220>
   <221> MISC_FEATURE
   <222> (11)..(11)
   <223> L-(paranitro)phénylalanine (Nop)
<220>
   <221> MISC_FEATURE
   <222> (14) .. (14)
   <223> L-prénylalanine (Pya)
<220>
   <221> MISC_FEATURE
   <222> (16)..(16)
   <223> Nle
<220>
   <221> MISC_FEATURE
   <222> (17)..(17)
   <223> L-leucine amidifiée
<400> 8
   Xaa Asn Lys Thr Arg Ile Asp Glu Ala Asn Xaa Arg Ala Xaa Lys Xaa
   1 5 10 15
   Xaa
<210> 9
   <211> 17
   <212> PRT
   <213> Séquence artificielle
<220>
   <223> Peptide dérivé de SNAP25
<220>
   <221> MISC_FEATURE
   <222> (1) .. (1)
   <223> L-sérine protégée par un reste Acétylcystéine
<220>
   <221> MISC_FEATURE
   <222> (11)..(11)
   <223> L-(paranitro)phénylalanine (Nop)
<220>
   <221> MISC_FEATURE
   <222> (14) .. (14)
   <223> L-prénylalanine (Pya)
<220>
   <221> MISC_FEATURE
   <222> (16)..(16)
   <223> Nle
<220>
   <221> MISC_FEATURE
   <222> (17)..(17)
   <223> L-leucine amidifiée
<400> 9
   Xaa Asn Lys Thr Arg Ile Asp Glu Ala Asn Xaa Arg Ala Xaa Lys Xaa
   1 5 10 15
   Xaa
<210> 10
   <211> 17
   <212> PRT
   <213> Séquence artificielle
<220>
   <223> Peptide dérivé de SNAP25
<220>
   <221> MISC_FEATURE
   <222> (1)..(1)
   <223> L-sérine acétylée
<220>
   <221> MISC_FEATURE
   <222> (11) .. (11)
   <223> L-(paranitro)phénylalanine (Nop)
<220>
   <221> MISC_FEATURE
   <222> (14)..(14)
   <223> L-prénylalanine (Pya)
<220>
   <221> MISC_FEATURE
   <222> (16) .. (16)
   <223> Nle
<220>
   <221> MISC_FEATURE
   <222> (17) .. (17)
<223> L-leucine amidifiée
<400> 10
   Xaa Asn Lys Thr Arg Ile Asp Gln Ala Asn Xaa Arg Ala Xaa Lys Xaa
   1               5                   10                  15
   Xaa
<210> 11
   <211> 17
   <212> PRT
   <213> Séquence artificielle
<220>
   <223> Peptide dérivé de SNAP25
<220>
   <221> MISC_FEATURE
   <222> (1)..(1)
   <223> L-sérine protégée par un reste Acétylcystéine
<220>
   <221> MISC_FEATURE
   <222> (11)..(11)
   <223> L-(paranitro)phénylalanine (Nop)
<220>
   <221> MISC_FEATUR.E
   <222> (14)..(14)
   <223> L-prénylalanine (Pya)
<220>
   <221> MISC_FEATURE
   <222> (16)..(16)
   <223> Nle
<220>
   <221> MISC_FEATURE
   <222> (17) .. (17)
   <223> L-leucine amidifiée
<400> 11
   Xaa Asn Lys Thr Arg Ile Asp Gln Ala Asn Xaa Arg Ala Xaa Lys Xaa
   1               5                   10                  15
   Xaa
<210> 12
   <211> 16
   <212> PRT
   <213> Séquence artificielle
<220>
   <223> Peptide dérivé de SNAP25
<220>
   <221> MISC_FEATURE
   <222> (1) .. (1)
   <223> L-sérine acétylée
<220>
   <221> MISC_FEATURE
   <222> (11)..(11)
   <223> L-(paranitro)phénylalanine (Nop)
<220>
   <221> MISC_FEATURE
   <222> (14)..(14)
   <223> L-prénylalanine (Pya)
<220>
   <221> MISC_FEATURE
   <222> (16)..(16)
   <223> Norleucine (Nle) amidifiée
<400> 12
   Xaa Asn Lys Thr Arg Ile Asp Gln Ala Asn Xaa Arg Ala Xaa Lys Xaa
   1 5 10 15
<210> 13
   <211> 16
   <212> PRT
   <213> Séquence artificielle
<220>
   <223> Peptide dérivé de SNAP25
<220>
   <221> MISC_FEATURE
   <222> (1)..(1)
   <223> L-sérine protégée par un reste Acétylcystéine
<220>
   <221> MISC_FEATURE
   <222> (11)..(11)
   <223> L-(paranitro)phénylalanine (Nop)
<220>
   <221> MISC_FEATURE
   <222> (14) .. (14)
   <223> L-prénylalanine (Pya)
<220>
   <221> MISC_FEATURE
   <222> (16)..(16)
   <223> Norleucine (Nle) amidifiée
<400> 13
   Xaa Asn Lys Thr Arg Ile Asp Gln Ala Asn Xaa Arg Ala Xaa Lys Xaa
   1 5 10 15
<210> 14
   <211> 23
   <212> PRT
   <213> Séquence artificielle
<220>
   <223> Peptide dérivé de SNAP25
<220>
   <221> MISC_FEATURE
   <222> (1)..(1)
   <223> L-lysine acétylée
<220>
   <221> MISC_FEATURE
   <222> (14) .. (14)
   <223> L-(paranitro)phénylalanine (Nop)
<220>
   <221> MISC_FEATURE
   <222> (17) .. (17)
   <223> L-prénylalanine (Pya)
<220>
   <221> MISC_FEATURE
   <222> (19)..(19)
   <223> Nle
<220>
   <221> MISC_FEATURE
   <222> (23)..(23)
   <223> L-glycine amidifiée
<400> 14
   Xaa Ser Asp Ser Asn Lys Thr Arg Ile Asp Glu Ala Asn Xaa Arg Ala
   1               5                   10                  15
   Xaa Lys Xaa Leu Gly Ser Xaa
               20
<210> 15
   <211> 52
   <212> PRT
   <213> Séquence artificielle
<220>
   <223> Peptide dérivé de SNAP25
<220>
   <221> MISC_FEATURE
   <222> (12) .. (12)
   <223> Met ou Nle
<220>
   <221> MISC_FEATURE
   <222> (29)..(29)
   <223> Met ou Nle
<220>
   <221> MISC_FEATURE
   <222> (33)..(33)
   <223> Met ou Nle
<220>
   <221> MISC_FEATURE
   <222> (48) .. (48)
   <223> Met ou Nle
<400> 15
   Arg Arg Val Thr Asn Asp Ala Arg Glu Asn Glu Xaa Asp Glu Asn Leu
   1               5                   10                  15
   Glu Gln Val Ser Gly Ile Ile Gly Asn Leu Arg His Xaa Ala Leu Asp
               20                  25                  30
   Xaa Gly Asn Glu Ile Asp Thr Gln Asn Arg Gln Ile Asp Arg Ile Xaa
           35                  40                  45
   Glu Lys Ala Asp
       50
<210> 16
   <211> 46
   <212> PRT
   <213> Séquence artificielle
<220>
   <223> Peptide dérivé de SNAP25
<220>
   <221> MISC_FEATURE
   <222> (6)..(6)
   <223> Met ou Nle
<220>
   <221> MISC_FEATURE
   <222> (23)..(23)
   <223> Met ou Nle
<220>
   <221> MISC_FEATURE
   <222> (27)..(27)
   <223> Met ou Nle
<220>
   <221> MISC_FEATURE
   <222> (42)..(42)
   <223> Met ou Nle
<400> 16
   Ala Arg Glu Asn Glu Xaa Asp Glu Asn Leu Glu Gln Val Ser Gly Ile
   1               5                   10                  15
   Ile Gly Asn Leu Arg His Xaa Ala Leu Asp Xaa Gly Asn Glu Ile Asp
               20                  25                  30
   Thr Gln Asn Arg Gln Ile Asp Arg Ile Xaa Glu Lys Ala Asp
           35                  40                  45
<210> 17
   <211> 41
   <212> PRT
   <213> Séquence artificielle
<220>
   <223> Peptide dérivé de SNAP25
<220>
   <221> MISC_FEATURE
   <222> (1) .. (1)
   <223> Met ou Nle
<220>
   <221> MISC_FEATURE
   <222> (18)..(18)
   <223> Met ou Nle
<220>
   <221> MISC_FEATURE
   <222> (22)..(22)
   <223> Met ou Nle
<220>
   <221> MISC_FEATURE
   <222> (37).. (37)
   <223> Met ou Nle
<400> 17
   Xaa Asp Glu Asn Leu Glu Gln Val Ser Gly Ile Ile Gly Asn Leu Arg
   1               5                   10                  15
   His Xaa Ala Leu Asp Xaa Gly Asn Glu Ile Asp Thr Gln Asn Arg Gln
               20                  25                  30
   Ile Asp Arg Ile Xaa Glu Lys Ala Asp
           35                  40
<210> 18
   <211> 31
   <212> PRT
   <213> Séquence artificielle
<220>
   <223> Peptide dérivé de SNAP25
<220>
   <221> MISC_FEATURE
   <222> (8)..(8)
   <223> Met ou Nle
<220>
   <221> MISC_FEATURE
   <222> (12) .. (12)
   <223> Met ou Nle
<220>
   <221> MISC_FEATURE
   <222> (27) .. (27)
   <223> Met ou Nle
<400> 18
   Ile Ile Gly Asn Leu Arg His Xaa Ala Leu Asp Xaa Gly Asn Glu Ile
   1               5                   10                  15
   Asp Thr Gln Asn Arg Gln Ile Asp Arg Ile Xaa Glu Lys Ala Asp
               20                  25                  30
<210> 19
   <211> 69
   <212> PRT
   <213> Séquence artificielle
<220>
   <223> Peptide dérivé de SNAP25
<220>
   <221> MISC_FEATURE
   <222> (1)..(1)
   <223> L-arginine acétylée
<220>
   <221> MISC_FEATURE
   <222> (63)..(63)
   <223> L-(paranitro)phénylalanine (Nop)
<220>
   <221> MISC_FEATURE
   <222> (66) .. (66)
   <223> L-prénylalanine (Pya)
<220>
   <221> MISC_FEATURE
   <222> (68)..(68)
   <223> Nle
<220>
   <221> MISC_FEATURE
   <222> (69)..(69)
   <223> L-leucine amidifiée
<400> 19
   Xaa Arg Val Thr Asn Asp Ala Arg Glu Asn Glu Met Asp Glu Asn Leu
   1               5                   10                  15
   Glu Gln Val Ser Gly Ile Ile Gly Asn Leu Arg His Met Ala Leu Asp
               20                  25                  30
   Met Gly Asn Glu Ile Asp Thr Gln Asn Arg Gln Ile Asp Arg Ile Met
           35                  40                  45
   Glu Lys Ala Asp Ser Asn Lys Thr Arg Ile Asp Glu Ala Asn Xaa Arg
       50                  55                  60
   Ala Xaa Lys Xaa Xaa
   65
<210> 20
   <211> 48
   <212> PRT
   <213> Séquence artificielle
<220>
   <223> Peptide dérivé de SNAP25
<220>
   <221> MISC_FEATURE
   <222> (1) .. (1)
   <223> L-isoleucine acétylée
<220>
   <221> MISC_FEATURE
   <222> (42)..(42)
   <223> L-(paranitro)phénylalanine (Nop)
<220>
   <221> MISC_FEATURE
   <222> (45) .. (45)
   <223> L-prénylalanine (Pya)
<220>
   <221> MISC_FEATURE
   <222> (47)..(47)
   <223> Nle
<220>
   <221> MISC_FEATURE
   <222> (48)..(48)
   <223> L-leucine amidifiée
<400> 20
   Xaa Ile Gly Asn Leu Arg His Met Ala Leu Asp Met Gly Asn Glu Ile
   1 5 10 15
   Asp Thr Gln Asn Arg Gln Ile Asp Arg Ile Met Glu Lys Ala Asp Ser
   20 25 30
   Asn Lys Thr Arg Ile Asp Glu Ala Asn Xaa Arg Ala Xaa Lys Xaa Xaa
   35 40 45
<210> 21
   <211> 48
   <212> PRT
   <213> Séquence artificielle
<220>
   <223> Peptide dérivé de SNAP25
<220>
   <221> MISC_FEATURE
   <222> (1) .. (1)
   <223> L-isoleucine acétylée
<220>
   <221> MISC_FEATURE
   <222> (8)..(8)
   <223> Nle
<220>
   <221> MISC_FEATURE
   <222> (12)..(12)
   <223> Nle
<220>
   <221> MISC_FEATURE
   <222> (27) .. (27)
   <223> Nle
<220>
   <221> MISC_FEATURE
   <222> (42)..(42)
   <223> L-(paranitro)phénylalanine (Nop)
<220>
   <221> MISC_FEATURE
   <222> (45)..(45)
   <223> L-prénylalanine (Pya)
<220>
   <221> MISC_FEATURE
   <222> (47)..(47)
   <223> Nle
<220>
   <221> MISC_FEATURE
   <222> (48)..(48)
   <223> L-leucine amidifiée
<400> 21
   Xaa Ile Gly Asn Leu Arg His Xaa Ala Leu Asp Xaa Gly Asn Glu Ile
   1 5 10 15
   Asp Thr Gln Asn Arg Gln Ile Asp Arg Ile Xaa Glu Lys Ala Asp Ser
   20 25 30
   Asn Lys Thr Arg Ile Asp Glu Ala Asn Xaa Arg Ala Xaa Lys Xaa Xaa
   35 40 45
<210> 22
   <211> 9
   <212> PRT
   <213> Séquence artificielle
<220>
   <223> Peptide dérivé de SNAP25
<220>
   <221> MISC_FEATURE
   <222> (3) .. (3)
   <223> L-prénylalanine (Pya)
<220>
   <221> MISC_FEATURE
   <222> (5) .. (5)
   <223> Nle ou Nle amidifiée si aucun acide aminé en position suivante
<220>
   <221> MISC_FEATURE
   <222> (6)..(6)
   <223> Leu ou Leu amidifiée si aucun acide aminé en position suivante ou aucun acide aminé
<220>
   <221> MISC_FEATURE
   <222> (7)..(9)
   <223> Gly-Ser-Gly amidifiée ou aucun acide aminé
<400> 22
   Arg Ala Xaa Lys Xaa Xaa Xaa Xaa Xaa
   1 5
<210> 23
   <211> 6
   <212> PRT
   <213> Séquence artificielle
<220>
   <223> Peptide dérivé de SNAP25
<220>
   <221> MISC_FEATURE
   <222> (3)..(3)
   <223> L-prénylalanine (Pya)
<220>
   <221> MISC_FEATURE
   <222> (5)..(5)
   <223> Nle
<220>
   <221> MISC_FEATURE
   <222> (6).. (6)
   <223> L-leucine amidifiée
<400> 23
   Arg Ala Xaa Lys Xaa Xaa
   1               5
<210> 24
   <211> 5
   <212> PRT
   <213> Séquence artificielle
<220>
   <223> Peptide dérivé de SNAP25
<220>
   <221> MISC_FEATURE
   <222> (3)..(3)
   <223> L-prénylalanine (Pya)
<220>
   <221> MISC_FEATURE
   <222> (5)..(5)
   <223> Norleucine (Nle) amidifiée
<400> 24
   Arg Ala Xaa Lys Xaa
   1               5
<210> 25
   <211> 9
   <212> PRT
   <213> Séquence artificielle
<220>
   <223> Peptide dérivé de SNAP25
<220>
   <221> MISC_FEATURE
   <222> (3)..(3)
   <223> L-prénylalanine (Pya)
<220>
   <221> MISC_FEATURE
   <222> (5)..(5)
   <223> Nle
<220>
   <221> MISC_FEATURE
   <222> (9)..(9)
   <223> Glycine amidifiée
<400> 25
   Arg Ala Xaa Lys Xaa Leu Gly Ser Xaa
   1               5
<210> 26
   <211> 42
   <212> PRT
   <213> Séquence artificielle
<220>
   <223> Peptide dérivé de SNAP25
<220>
   <221> MISC_FEATURE
   <222> (1)..(1)
   <223> L-isoleucine amidifiée
<220>
   <221> MISC_FEATURE
   <222> (42)..(42)
   <223> L-(paranitro)phénylalanine (Nop)
<400> 26
   Xaa Ile Gly Asn Leu Arg His Met Ala Leu Asp Met Gly Asn Glu Ile
   1               5                   10                  15
   Asp Thr Gln Asn Arg Gln Ile Asp Arg Ile Met Glu Lys Ala Asp Ser
               20                  25                  30
   Asn Lys Thr Arg Ile Asp Glu Ala Asn Xaa
           35                  40
<210> 27
   <211> 17
   <212> PRT
   <213> Séquence artificielle
<220>
   <223> Peptide dérivé de SNAP25
<220>
   <221> MISC_FEATURE
   <222> (1)..(1)
   <223> L-sérine acétylée
<220>
   <221> MISC_FEATURE
   <222> (11) .. (11)
   <223> L-prénylalanine (Pya)
<220>
   <221> MISC_FEATURE
   <222> (14) .. (14)
   <223> L-(paranitro)phénylalanine (Nop)
<220>
   <221> MISC_FEATURE
   <222> (16) .. (16)
   <223> Nle
<220>
   <221> MISC_FEATURE
   <222> (17)..(17)
   <223> L-leucine amidifiée
<400> 27
   Xaa Asn Lys Thr Arg Ile Asp Glu Ala Asn Xaa Arg Ala Xaa Lys Xaa
   1               5                   10                  15
   Xaa

### LISTE DE SEQUENCES

<110> PHARMALEADS
<120> SUBSTRAT PEPTIDIQUE RECONNU PAR LA TOXINE BOTULIQUE DE TYPE A, BONT/A ET SON UTILISATION POUR DETECTER ET/OU DOSER LADITE TOXINE OU DES INHIBITEURS DE SON ACTIVITE
<130> BOTA
<160> 27
<170> PatentIn version 3.1
<210> 1
   <211> 206
   <212> PRT
   <213> Homo sapiens
<400> 1
<210> 2
   <211> 17
   <212> PRT
   <213> Séquence artificielle
<220>
   <223> Peptide dérivé de SNAP25
<400> 2
   Ser Asn Lys Thr Arg Ile Asp Glu Ala Asn Gln Arg Ala Thr Lys Met
   1 5 10 15
   Leu
<210> 3
   <211> 4
   <212> PRT
   <213> Séquence artificielle
<220>
   <223> Peptide dérivé de SNAP25
<220>
   <221> MISC_FEATURE
   <222> (1)..(1)
   <223> L-(paranitro)phénylalaniné (Nop)
<220>
   <221> MISC_FEATURE
   <222> (4)..(4)
   <223> L-prénylalanine (Pya)
<400> 3
   Xaa Arg Ala Xaa
   1
<210> 4
   <211> 5
   <212> PRT
   <213> Séquence artificielle
<220>
   <223> Peptide dérivé de SNAP25
<220>
   <221> MISC_FEATURE
   <222> (1)..(1)
   <223> L-(paranitro)phénylalanine (Nop)
<220>
   <221> MISC_FEATURE
   <222> (5)..(5)
   <223> L-prénylalanine (Pya)
<400> 4
   Xaa Arg Ala Thr Xaa
   1 5
<210> 5
   <211> 6
   <212> PRT
   <213> Séquence artificielle
<220>
   <223> Peptide dérivé de SNAP25
<220>
   <221> MISC_FEATURE
   <222> (1)..(1)
   <223> L-(paranitro)phénylalanine (Nop)
<220>
   <221> MISC_FEATURE
   <222> (6) .. (6)
   <223> L-prénylalanine (Pya).
<400> 5
   Xaa Arg Ala Thr Lys Xaa
   1               5
<210> 6
   <211> 21
   <212> PRT
   <213> Séquence artificielle
<220>
   <223> Peptide dérivé de SNAP25
<220>
   <221> MISC_FEATURE
   <222> (1)..(1)
   <223> Un acide aminé acétylé ou un enchaînement de 2 à 52 acides aminés acétylé du côté N-terminal ou aucun acide aminé
<220>
   <221> MISC_FEATURE
   <222> (2)..(2)
   <223> Ser ou Ser acétylée si aucun acide aminé du côté N-terminal
<220>
   <221> MISC_FEATURE
   <222> (9)..(9)
   <223> Glu ou Gln
<220>
   <221> MISC_FEATURE
   <222> (12) .. (12)
   <223> L-(paranitro)phénylalanine (Nop)
<220>
   <221> MISC_FEATURE
   <222> (15)..(15)
   <223> L-prénylalanine (Pya)
<220>
   <221> MISC_FEATURE
   <222> (17)..(17)
   <223> Nle ou Nle amidifiée si aucun acide aminé en position suivante
<220>
   <221> MISC_FEATURE
   <222> (18)..(18)
   <223> L-leucine ou L-leucine amidifiée si aucun acide aminé en position suivante ou aucun acide aminé
<220>
   <221> MISC_FEATURE
   <222> (19)..(21)
   <223> Gly-Ser-Gly amidifié ou aucun acide aminé
<400> 6
   Xaa Xaa Asn Lys Thr Arg Ile Asp Xaa Ala Asn Xaa Arg Ala Xaa Lys
   1               5                   10                  15
   Xaa Xaa Xaa Xaa Xaa
               20
<210> 7
   <211> 18
   <212> PRT
   <213> Séquence artificielle
<220>
   <223> Séquence dérivée de SNAP25
<220>
   <221> MISC_FEATURE
   <222> (1)..(1)
   <223> L-aspartate acétylée ou L-cystéine acétylée ou aucun acide aminé
<220>
   <221> MISC_FEATURE
   <222> (2) .. (2)
   <223> L-sérine ou L-sérine acétylée si aucun acide aminé en position précédente
<220>
   <221> MISC_FEATURE
   <222> (9)..(9)
   <223> Glu ou Gln
<220>
   <221> MISC_FEATURE
   <222> (12) .. (12)
   <223> L-(paranitro)phénylalanine (Nop)
<220>
   <221> MISC_FEATURE
   <222> (15)..(15)
   <223> L-prénylalanine (Pya)
<220>
   <221> MISC_FEATURE
   <222> (17) .. (17)
   <223> Nle ou Nle amidifiée si aucun acide aminé en position 18
<220>
   <221> MISC_FEATURE
   <222> (18)..(18)
   <223> L-leucine amidifiée ou aucun acide aminé
<400> 7
   Xaa Xaa Asn Lys Thr Arg Ile Asp Xaa Ala Asn Xaa Arg Ala Xaa Lys
   1               5                   10                  15
   Xaa Xaa
<210> 8
   <211> 17
   <212> PRT
   <213> Séquence artificielle
<220>
   <223> Peptide dérivé de SNAP25
<220>
   <221> MISC_FEATURE
   <222> (1)..(1)
   <223> L-sérine acétylée
<220>
   <221> MISC_FEATURE
   <222> (11)..(11)
   <223> L-(paranitro)phénylalanine (Nop)
<220>
   <221> MISC_FEATURE
   <222> (14)..(14)
   <223> L-prénylalanine (Pya)
<220>
   <221> MISC_FEATURE
   <222> (16)..(16)
   <223> Nle
<220>
   <221> MISC_FEATURE
   <222> (17)..(17)
   <223> L-leucine amidifiée
<400> 8
   Xaa Asn Lys Thr Arg Ile Asp Glu Ala Asn Xaa Arg Ala Xaa Lys Xaa
   1 5 10 15
   Xaa
<210> 9
   <211> 17
   <212> PRT
   <213> Séquence artificielle
<220>
   <223> Peptide dérivé de SNAP25
<220>
   <221> MISC_FEATURE
   <222> (1)..(1)
   <223> L-sérine protégée par un reste Acétylcystéine
<220>
   <221> MISC_FEATURE
   <222> (11)..(11)
   <223> L-(paranitro)phénylalanine (Nop)
<220>
   <221> MISC_FEATURE
   <222> (14)..(14)
   <223> L-prénylalanine (Pya)
<220>
   <221> MISC_FEATURE
   <222> (16) .. (16)
   <223> Nle
<220>
   <221> MISC_FEATURE
   <222> (17) .. (17)
   <223> L-leucine amidifiée
<400> 9
   Xaa Asn Lys Thr Arg Ile Asp Glu Ala Asn Xaa Arg Ala Xaa Lys Xaa
   1 5 10 15
   Xaa
<210> 10
   <211> 17
   <212> PRT
   <213> Séquence artificielle
<220>
   <223> Peptide dérivé de SNAP25
<220>
   <221> MISC_FEATURE
   <222> (1)..(1)
   <223> L-sérine acétylée
<220>
   <221> MISC_FEATURE
   <222> (11)..(11)
   <223> L-(paranitro)phénylalanine (Nop)
<220>
   <221> MISC_FEATURE
   <222> (14)..(14)
   <223> L-prénylalanine (Pya)
<220>
   <221> MISC_FEATURE
   <222> (16)..(16)
   <223> Nle
<220>
   <221> MISC_FEATURE
   <222> (17) .. (17)
   <223> L-leucine amidifiée
<400> 10
   Xaa Asn Lys Thr Arg Ile Asp Gln Ala Asn Xaa Arg Ala Xaa Lys Xaa
   1               5                   10                  15
   Xaa
<210> 11
   <211> 17
   <212> PRT
   <213> Séquence artificielle
<220>
   <223> Peptide dérivé de SNAP25
<220>
   <221> MISC_FEATURE
   <222> (1)..(1)
   <223> L-sérine protégée par un reste Acétylcystéine
<220>
   <221> MISC_FEATURE
   <222> (11)..(11)
   <223> L-(paranitro)phénylalanine (Nop)
<220>
   <221> MISC_FEATURE
   <222> (14)..(14)
   <223> L-prénylalanine (Pya)
<220>
   <221> MISC_FEATURE
   <222> (16)..(16)
   <223> Nle
<220>
   <221> MISC_FEATURE
   <222> (17)..(17)
   <223> L-leucine amidifiée
<400> 11
   Xaa Asn Lys Thr Arg Ile Asp Gln Ala Asn Xaa Arg Ala Xaa Lys Xaa
   1               5                   10                  15
   Xaa
<210> 12
   <211> 16
   <212> PRT
   <213> Séquence artificielle
<220>
   <223> Peptide dérivé de SNAP25
<220>
   <221> MISC_FEATURE
   <222> (1)..(1)
   <223> L-sérine acétylée
<220>
   <221> MISC_FEATURE
   <222> (11)..(11)
   <223> L-(paranitro)phénylalanine (Nop)
<220>
   <221> MISC_FEATURE
   <222> (19)..(14)
   <223> L-prénylalanine (Pya)
<220>
   <221> MISC_FEATURE
   <222> (16)..(16)
   <223> Norleucine (Nle) amidifiée
<400> 12
   Xaa Asn Lys Thr Arg Ile Asp Gln Ala Asn Xaa Arg Ala Xaa Lys Xaa
   1               5                   10                  15
<210> 13
   <211> 16
   <212> PRT
   <213> Séquence artificielle
<220>
   <223> Peptide dérivé de SNAP25
<220>
   <221> MISC_FEATURE
   <222> (1)..(1)
   <223> L-sérine protégée par un reste Acétylcystéine
<220>
   <221> MISC_FEATURE
   <222> (11)..(11)
   <223> L-(paranitro)phénylalanine (Nop)
<220>
   <221> MISC_FEATURE
   <222> (14)..(14)
   <223> L-prénylalanine (Pya)
<220>
   <221> MISC_FEATURE
   <222> (16)..(16)
   <223> Norleucine (Nle) amidifiée
<400> 13
   Xaa Asn Lys Thr Arg Ile Asp Gln Ala Asn Xaa Arg Ala Xaa Lys Xaa
   1               5                   10                  15
<210> 14
   <211> 23
   <212> PRT
   <213> Séquence artificielle
<220>
   <223> Peptide dérivé de SNAP25
<220>
   <221> MISC_FEATURE
   <222> (1)..(1)
   <223> L-lysine acétylée
<220>
   <221> MISC_FEATURE
   <222> (14)..(14)
   <223> L-(paranitro)phénylalanine (Nop)
<220>
   <221> MISC_FEATURE
   <222> (17) .. (17)
   <223> L-prénylalanine (Pya)
<220>
   <221> MISC_FEATURE
   <222> (19)..(19)
   <223> Nle
<220>
   <221> MISC_FEATURE
   <222> (23)..(23)
   <223> L-glycine amidifiée
<400> 14
   Xaa Ser Asp Ser Asn Lys Thr Arg Ile Asp Glu Ala Asn Xaa Arg Ala
   1               5                   10                  15
   Xaa Lys Xaa Leu Gly Ser Xaa
               20
<210> 15
   <211> 52
   <212> PRT
   <213> Séquence artificielle
<220>
   <223> Peptide dérivé de SNAP25
<220>
   <221> MISC_FEATURE
   <222> (12)..(12)
   <223> Met ou Nle
<220>
   <221> MISC_FEATURE
   <222> (29)..(29)
   <223> Met ou Nle
<220>
   <221> MISC_FEATURE
   <222> (33)..(33)
   <223> Met ou Nle
<220>
   <221> MISC_FEATURE
   <222> (48)..(48)
   <223> Met ou Nle
<400> 15
   Arg Arg Val Thr Asn Asp Ala Arg Glu Asn Glu Xaa Asp Glu Asn Leu
   1               5                   10                  15
   Glu Gln Val Ser Gly Ile Ile Gly Asn Leu Arg His Xaa Ala Leu Asp
               20                  25                  30
   Xaa Gly Asn Glu Ile Asp Thr Gln Asn Arg Gln Ile Asp Arg Ile Xaa
           35                  40                  45
   Glu Lys Ala Asp
       50
<210> 16
   <211> 46
   <212> PRT
   <213> Séquence artificielle
<220>
   <223> Peptide dérivé de SNAP25
<220>
   <221> MISC_FEATURE
   <222> (6)..(6)
   <223> Met ou Nle
<220>
   <221> MISC_FEATURE
   <222> (23)..(23)
   <223> Met ou Nle
<220>
   <221> MISC_FEATURE
   <222> (27) .. (27)
   <223> Met ou Nle
<220>
   <221> MISC_FEATURE
   <222> (42)..(42)
   <223> Met ou Nle
<400> 16
   Ala Arg Glu Asn Glu Xaa Asp Glu Asn Leu Glu Gln Val Ser Gly Ile
   1               5                   10                  15
   Ile Gly Asn Leu Arg His Xaa Ala Leu Asp Xaa Gly Asn Glu Ile Asp
               20                  25                  30
   Thr Gln Asn Arg Gln Ile Asp Arg Ile Xaa Glu Lys Ala Asp
           35                  40                  45
<210> 17
   <211> 41
   <212> PRT
   <213> Séquence artificielle
<220>
   <223> Peptide dérivé de SNAP25
<220>
   <221> MISC_FEATURE
   <222> (1) .. (1)
   <223> Met ou Nle
<220>
   <221> MISC_FEATURE
   <222> (18)..(18)
   <223> Met ou Nle
<220>
   <221> MISC_FEATURE
   <222> (22)..(22)
   <223> Met ou Nle
<220>
   <221> MISC_FEATURE
   <222> (37)..(37)
   <223> Met ou Nle
<400> 17
   Xaa Asp Glu Asn Leu Glu Gln Val Ser Gly Ile Ile Gly Asn Leu Arg
   1               5                   10                  15
   His Xaa Ala Leu Asp Xaa Gly Asn Glu Ile Asp Thr Gln Asn Arg Gln
               20                  25                  30
   Ile Asp Arg Ile Xaa Glu Lys Ala Asp
           35                  40
<210> 18
   <211> 31
   <212> PRT
   <213> Séquence artificielle
<220>
   <223> Peptide dérivé de SNAP25
<220>
   <221> MISC_FEATURE
   <222> (8)..(8)
   <223> Met ou Nle
<220>
   <221> MISC_FEATURE
   <222> (12)..(12)
   <223> Met ou Nle
<220>
   <221> MISC_FEATURE
   <222> (27) .. (27)
   <223> Met ou Nle
<400> 18
   Ile Ile Gly Asn Leu Arg His Xaa Ala Leu Asp Xaa Gly Asn Glu Ile
   1               5                   10                  15
   Asp Thr Gln Asn Arg Gln Ile Asp Arg Ile Xaa Glu Lys Ala Asp
               20                  25                  30
<210> 19
   <211> 69
   <212> PRT
   <213> Séquence artificielle
<220>
   <223> Peptide dérivé de SNAP25
<220>
   <221> MISC_FEATURE
   <222> (1)..(1)
   <223> L-arginine acétylée
<220>
   <221> MISC_FEATURE
   <222> (63)..(63)
   <223> L-(paranitro)phénylalanine (Nop)
<220>
   <221> MISC_FEATURE
   <222> (66)..(66)
   <223> L-prénylalanine (Pya)
<220>
   <221> MISC_FEATURE
   <222> (68) .. (68)
   <223> Nle
<220>
   <221> MISC_FEATURE
   <222> (69)..(69)
   <223> L-leucine amidifiée
<400> 19
   Xaa Arg Val Thr Asn Asp Ala Arg Glu Asn Glu Met Asp Glu Asn Leu
   1               5                   10                  15
   Glu Gln Val Ser Gly Ile Ile Gly Asn Leu Arg His Met Ala Leu Asp
               20                  25                  30
   Met Gly Asn Glu Ile Asp Thr Gln Asn Arg Gln Ile Asp Arg Ile Met
           35                  40                  45
   Glu Lys Ala Asp Ser Asn Lys Thr Arg Ile Asp Glu Ala Asn Xaa Arg
       50                  55                  60
   Ala Xaa Lys Xaa Xaa
   65
<210> 20
   <211> 48
   <212> PRT
   <213> Séquence artificielle
<220>
   <223> Peptide dérivé de SNAP25
<220>
   <221> MISC_FEATURE
   <222> (1) .. (1)
   <223> L-isoleucine acétylée
<220>
   <221> MISC_FEATURE
   <222> (42)..(42)
   <223> L-(paranitro)phénylalanine (Nop)
<220>
   <221> MISC_FEATURE
   <222> (45)..(45)
   <223> L-prénylalanine (Pya)
<220>
   <221> MISC_FEATURE
   <222> (47)..(47)
   <223> Nle
<220>
   <221> MISC_FEATURE
   <222> (48)..(48)
   <223> L-leucine amidifiée
<400> 20
   Xaa Ile Gly Asn Leu Arg His Met Ala Leu Asp Met Gly Asn Glu Ile
   1               5                   10                  15
   Asp Thr Gln Asn Arg Gln Ile Asp Arg Ile Met Glu Lys Ala Asp Ser
               20                  25                  30
   Asn Lys Thr Arg Ile Asp Glu Ala Asn Xaa Arg Ala Xaa Lys Xaa Xaa
           35                  40                  45
<210> 21
   <211> 48
   <212> PRT
   <213> Séquence artificielle
<220>
   <223> Peptide dérivé de SNAP25
<220>
   <221> MISC_FEATURE
   <222> (1)..(1)
   <223> L-isoleucine acétylée
<220>
   <221> MISC_FEATURE
   <222> (8) .. (8)
   <223> Nle
<220>
   <221> MISC_FEATURE
   <222> (12) .. (12)
   <223> Nle
<220>
   <221> MISC_FEATURE
   <222> (27)..(27)
   <223> Nle
<220>
   <221> MISC_FEATURE
   <222> (42) .. (42)
   <223> L-(paranitro)phénylalanine (Nop)
<220>
   <221> MISC_FEATURE
   <222> (45)..(45)
   <223> L-prénylalanine (Pya)
<220>
   <221> MISC_FEATURE
   <222> (47)..(47)
   <223> Nle
<220>
   <221> MISC_FEATURE
   <222> (48)..(98)
   <223> L-leucine amidifiée
<400> 21
   Xaa Ile Gly Asn Leu Arg His Xaa Ala Leu Asp Xaa Gly Asn Glu Ile
   1 5 10 15
   Asp Thr Gln Asn Arg Gln Ile Asp Arg Ile Xaa Glu Lys Ala Asp Ser
   20 25 30
   Asn Lys Thr Arg Ile Asp Glu Ala Asn Xaa Arg Ala Xaa Lys Xaa Xaa
   35 40 45
<210> 22
   <211> 9
   <212> PRT
   <213> Séquence artificielle
<220>
   <223> Peptide dérivé de SNAP25
<220>
   <221> MISC_FEATURE
   <222> (3)..(3)
   <223> L-prénylalanine (Pya)
<220>
   <221> MISC_FEATURE
   <222> (5)..(5)
   <223> Nle ou Nle amidifiée si aucun acide aminé en position suivante
<220>
   <221> MISC_FEATURE
   <222> (6)..(6)
   <223> Leu ou Leu amidifiée si aucun acide aminé en position suivante ou aucun acide aminé
<220>
   <221> MISC_FEATURE
   <222> (7)..(9)
   <223> Gly-Ser-Gly amidifiée ou aucun acide aminé
<400> 22
   Arg Ala Xaa Lys Xaa Xaa Xaa Xaa Xaa
   1               5
<210> 23
   <211> 6
   <212> PRT
   <213> Séquence artificielle
<220>
   <223> Peptide dérivé de SNAP25
<220>
   <221> MISC_FEATURE
   <222> (3)..(3)
   <223> L-prénylalanine (Pya)
<220>
   <221> MISC_FEATURE
   <222> (5)..(5)
   <223> Nle
<220>
   <221> MISC_FEATURE
   <222> (6) .. (6)
   <223> L-leucine amidifiée
<400> 23
   Arg Ala Xaa Lys Xaa Xaa
   1               5
<210> 24
   <211> 5
   <212> PRT
   <213> Séquence artificielle
<220>
   <223> Peptide dérivé de SNAP25
<220>
   <221> MISC_FEATURE
   <222> (3)..(3).
   <223> L-prénylalanine (Pya)
<220>
   <221> MISC_FEATURE,
   <222> (5)..(5)
   <223> Norleucine (Nle) amidifiée
<400> 24
   Arg Ala Xaa Lys Xaa
   1               5
<210> 25
   <211> 9
   <212> PRT
   <213> Séquence artificielle
<220>
   <223> Peptide dérivé de SNAP25
<220>
   <221> MISC_FEATURE
   <222> (3)..(3)
   <223> L-prénylalanine (Pya)
<220>
   <221> MISC_FEATURE
   <222> (5) .. (5)
   <223> Nle
<220>
   <221> MISC_FEATURE
   <222> (9) .. (9)
   <223> Glycine amidifiée
<400> 25
   Arg Ala Xaa Lys Xaa Leu Gly Ser Xaa
   1               5
<210> 26
   <211> 42
   <212> PRT
   <213> Séquence artificielle
<220>
   <223> Peptide dérivé de SNAP25
<220>
   <221> MISC_FEATURE
   <222> (1)..(1)
   <223> L-isoleucine amidifiée
<220>
   <221> MISC_FEATURE
   <222> (42)..(42)
   <223> L-(paranitro)phénylalanine (Nop)
<400> 26
   Xaa Ile Gly Asn Leu Arg His Met Ala Leu Asp Met Gly Asn Glu Ile
   1 5 10 15
   Asp Thr Gln Asn Arg Gln Ile Asp Arg Ile Met Glu Lys Ala Asp Ser
   20 25 30
   Asn Lys Thr Arg Ile Asp Glu Ala Asn Xaa
   35 40
<210> 27
   <211> 17
   <212> PRT
   <213> Séquence artificielle
<220>
   <223> Peptide dérivé de SNAP25
<220>
   <221> MISC_FEATU-RE
   <222> (1)..(1)
   <223> L-sérine acétylée
<220>
   <221> MISC_FEATURE
   <222> (11)..(11)
   <223> L-prénylalanine (Pya)
<220>
   <221> MISC_FEATURE
   <222> (14)..(14)
   <223> L-(paranitro)phénylalanine (Nop)
<220>
   <221> MISC_FEATURE
   <222> (16)..(16)
   <223> Nle
<220>
   <221> MISC_FEATURE
   <222> (17) .. (17)
   <223> L-leucine amidifiée
<400> 27
   Xaa Asn Lys Thr Arg Ile Asp Glu Ala Asn Xaa Arg Ala Xaa Lys Xaa
   1 5 10 15
   Xaa

## Description

La présente invention a pour objet des substrats peptidiques reconnus sélectivement par la toxine botulique de type A, BoNT/A, et leurs utilisations, notamment dans le cadre de la mise en oeuvre de procédés pour détecter, identifier et/ou doser la toxine botulique de type A, ou des inhibiteurs et/ou activateurs de l'activité métallopeptidasique de ladite toxine.

La neurotoxine de type A (BoNT/A) fait partie d'une famille de sept protéines apparentées (toxines botuliques A à G) produites par différentes souches du bacille anaérobie « clostridium botulinum ». Les deux formes les plus fréquemment rencontrées et les plus dangereuses sont les toxines de type A et B. Les doses létales 50 chez la souris par voie intrapéritonéale sont de 2 pg pour la toxine A et d'environ 50 à 100 pg pour la toxine B. Elles agissent au niveau du système nerveux périphérique de l'homme et de diverses espèces animales en induisant le « botulisme » caractérisé par une paralysie flasque des muscles du squelette pouvant entraîner la mort.

La forme majeure d'intoxication par ces toxines est due à l'ingestion de nourriture ou boissons contaminées. Mais ces toxines peuvent également constituer une arme biologique potentielle dans la mesure où elles sont faciles à produire. D'autre part, depuis plusieurs années, les toxines botuliques, et plus particulièrement la BoNT/A, sont utilisées pour des applications thérapeutiques (dystonies, hyperactivités neuronales telles que strabisme, blépharospasme etc..) ou esthétiques (notamment la réduction des rides). Pour toutes ces applications, il est indispensable de posséder une méthode simple, rapide et sensible, de détection et de quantification de la toxine botulique de type A dans divers milieux, y compris biologiques.

Les neurotoxines botuliques sont constituées de deux sous unités protéiques : une chaîne lourde (100 kD) associée à une chaîne légère (50 kD) par l'intermédiaire d'un pont disulfure. La chaîne lourde intervient dans la liaison de la toxine à la terminaison nerveuse, dans l'intemalisation puis dans la translocation de la chaîne légère dans le cytosol. La chaîne légère est responsable de la toxicité de la protéine par inhibition de la libération, calcium dépendante, de l'acétylcholine.

La toxicité de la chaîne légère de ces toxines est due à son activité peptidasique. En effet, les toxines botuliques appartiennent à la famille des métallopeptidases à zinc et plus particulièrement à la sous-famille des zincines qui contient la séquence consensus HExxH; (Schiavo et al. (1992) J. Biol. Chem. 267, 23479-23483 ; Roques B.P. (1993) Biochem. Soc. Trans. 21, 678-685).

Elles clivent de façon très spécifique les protéines neuronales impliquées dans l'exocytose des neurotransmetteurs, telles que la SNAP 25 et la synaptobrévine. Le site de clivage est spécifique pour chaque toxine y compris pour un substrat identique.

La toxine botulique de type A clive spécifiquement une des protéines du complexe SNARE, le SNAP-25 (séquence SEQ ID NO : 1). Cette protéine de 206 aminoacides est clivée spécifiquement au niveau de la liaison Q197-R198.

Des travaux antérieurs ont montré que des fragments plus courts que cette séquence SNAP 25 (1-206) pouvaient également être dégradés par la BoNT/A (Schmidt et Bostian, J. Prot. Chem.,1997,16,19-26) . Il en est ainsi du fragment minimum SNAP 25 (187-203) : ¹⁸⁷ S N K T R I D E A N R A T K M L ²⁰³ (séquence SEQ ID NO : 2).

L'approche la plus efficace pour combattre les effets néfastes de la BoNT/A, soit au cours d'un botulisme déclaré, soit au cours de contre-indications thérapeutiques, est le développement d'inhibiteurs sélectifs et de haute affinité pour son activité métallopeptidasique, responsable de sa toxicité. Toutefois, l'identification de tels inhibiteurs, et la détermination de leur efficacité (constantes d'inhibition, Ki), exigent un test simple et robotisable de mesure de l'activité BoNT/A permettant un grand nombre d'essais (HTS).

Les tests actuellement disponibles ne sont pas satisfaisants pour de telles applications.

D'autre part, la méthode de détection la plus sensible des toxines botuliques est un essai *in vivo* qui repose sur la détermination de la dose létale 50 chez la souris (DL 50) (Kautter et Salomon (1976) J. Assoc. Anal. Chem., 60, 541-545). Bien que très sensible, puisqu'elle permet de détecter de 5 à 10 pg de BoNT/A, cette méthode présente de nombreux inconvénients : le temps de réponse est de plusieurs jours et elle ne paraît pas être la méthode la plus précise pour estimer l'activité biologique d'une préparation thérapeutique de toxine (Pearce et al. (1994) Tox.Applied Pharmacol., 128, 69-77). De plus, l'estimation de la DL50 varie très largement avec la préparation de toxine, la souche de souris, etc. Enfin notons que l'expérimentation sur animal est de plus en plus controversée.

Des tests sur lignées cellulaires ont été proposés (De Waart et al. (1972) Zentralblatt fur Bacteriology, 222, 96-114), mais leur sensibilité est trop faible (1000 DL50 souris). Des tests immunologiques ont été développés [radioimmunoessais (Boroff et Shu-Chen, 1973, Applied Microbiol., 25, 545-549), tests ELISA (Hallis et al., 1993 in Botulinum and Tetanus Toxins DasGupta B.R. editeur, New York : Plenum Press) avec des systèmes d'amplification (Stanley et al. ,1985,J. Immunol. and Methods, 83, 89-95)], mais aucun n'est suffisamment sensible. Par ailleurs, ils donnent naissance à une proportion significative de faux positifs.

Quelques essais, basés sur l'activité peptidasique de la BoNT/A ont été développés. D'une part, l'étude du clivage du SNAP 25 ou de son fragment SNAP 25(137-206) (Hallis et al. (1996) J. Clinic.Microbiol., 34, 1934-1938) immobilisé sur résine, suivie de la détection du métabolite fixé sur la résine par un anticorps, puis d'un système d'amplification, a conduit à la mise en évidence d'environ 1000 DL50 souris/ml. Néanmoins cette méthode est longue et coûteuse.

La caractérisation par Schmidt et Bostian (J. Prot. Chem. (1997), 16, 19-26) du plus petit fragment de SNAP 25, reconnu par la BoNT/A, (187-203) SNAP 25, a conduit à la mise au point du premier substrat fluorigénique de BoNT/A par introduction d'un groupe dnp (dinitrophenyl) et DACIA (diméthyl-amino-coumarinyl) sur les chaînes latérales d'une lysine en position 197 et d'une cystéine en position 200, respectivement (Schmidt et Stafford, Applied Environ. Microbiol. (2003), 69, 297-303). Cette méthode permet de détecter rapidement des quantités supérieures ou égales à 100ng/mL.

Néanmoins, toutes ces méthodes ne possèdent pas la sensibilité, la reproductibilité, la fiabilité et la facilité d'utilisation requises pour détecter et quantifier les doses de BoNT/A native inférieures ou égales à 1 ng/ml, la concentration correspondant à la dose létale pour un homme de 70 kg étant de l'ordre de 1 µg. Ceci implique de délecter des doses inférieures à 5 ng dans un petit volume de milieu infecté par la BoNT/A, en cas d'ingestion.

La présente invention a précisément pour objet de proposer un nouveau test de détection et de quantification de la BoNT/A permettant de répondre à ces impératifs.

La méthode choisie pour le dosage de la BoNT/A dans le cadre de la présente invention repose sur le phénomène d'extinction de fluorescence par collision entre un fluorophore (L.pyrenyl-alanine, Pya) et un élément très polaire (L.p-nitro-phénylalanine, NOP) (substrat à fluorescence réprimée selon un mécanisme de répression par collision ; brevet FR 0004507). A l'inverse du FRET (fluorescence par transfert d'énergie), il n'existe aucun recouvrement du spectre d'émission de fluorescence du reste L-Pya et du spectre d'absorption de L-NOP. La pyrénylalanine Pya présente une fluorescence très importante qui est quasi totalement éteinte lorsque Pya est placé dans un enchaînement peptidique à proximité du reste Nop. En conséquence, la fluorescence de Pya ne peut se manifester qu'à partir du moment où la séquence peptidique située entre Pya et Nop est clivée.

L'extinction de fluorescence de Pya en présence de NOP est due à l'établissement lors de l'excitation par irradiation d'un nouvel état excité différent de celui de Pya en absence de NOP. Le nouvel état se désactive très facilement et rapidement par conversion interne de l'énergie et ce sans émission (non-radiative process).

La méthode choisie dans le cadre de la présente invention se distingue donc très nettement de cette utilisée dans le FRET. Dans ce dernier cas, comme indiqué précédemment, le recouvrement des spectres d'absorption de l'accepteur (quencher) et d'émission du donneur (fluorophore) créé un nouvel état excité différent de celui créé en absence de l'accepteur. Cet état se désactive très facilement par conversion interne de l'énergie et ce, sans émission (non radiative process). L'aspect théorique de ces méthodes est rapportée dans: Yaron et al., Anal. Biochem. 95, 228-235 (1979) et Lakowicz J.R., Principles of fluorescence spectroscopy, 2eme ed., Ed. KA.PP.

Dans les deux méthodes, l'extinction de fluorescence est dépendante de la distance entre les deux fluorophores. L'efficacité des deux procédés peut être étudiée expérimentalement sur des exemples précis où les configurations (distances entre les deux partenaires) sont optimales. C'est le cas des molécules suivantes dans lesquelles les entités impliquées ont été introduites côte-à-côte.

### FRET

### ---DABCYL-CO-↑-NH-EDANS-

DABCYL = 4-[4(dimethylaminopenyl-azo)benzoic acid]
EDANS = Ethylène-DiAmino-Naphtalène-Sulfonate.

L'hydrolyse enzymatique de la liaison CONH disperse théoriquement à l'infini les deux partenaires DABCYL et EDANS. L'augmentation de fluorescence observée de EDANS dans ce cas est de l'ordre de 200 UA (unité arbitraire) [G.T. Wang et al., Tet. Lett. 31, (1990)].

### Complexe de collision

### --- Pya - CO -↑- NH - Nop ---

L'augmentation de fluorescence de Pya lors de la coupure enzymatique de la liaison peptidique CONH conduit à une exaltation de fluorescence de 800 UA. [N. Luciani et al., Biochem. J. 356 (2001) ; brevet Fr 00.04507, PCT/FR01/01058].

Cette meilleure exaltation de fluorescence observée avec l'utilisation du complexe de collision Pya/Nop, est à l'origine du choix des couples Pya-Nop ou Nop-Pya par les inventeurs, et non du FRET.

Les inventeurs ont précédemment décrit un substrat très spécifique de la toxine botulique de type B dans lequel des résidus Pya et Nop ont été introduits en positions 74 et 77 respectivement du fragment 60-94 de la synaptobrévine, substrat naturel minimal de la BoNT/B (brevet FR 0007113; C. Anne et al. Analytical Biochem. (2001), 291, 2S3-261).

Dans le cas de la BoNT/A, le substrat naturel est le SNAP 25, et la séquence minimale du SNAP 25 clivée par la BoNT/A est le fragment (187-203)SNAP 25 (Schmidt et Bostian, supra) correspondant à la formule (187-203)SNAP25 = ¹⁸⁷S-N-K-T-R-I-D-E-A-N-Q¹⁹⁷R-A-T-K-M-L²⁰³ (SEQ ID NO : 2) indiquée ci-dessus.

Le clivage de ce peptide, ou du peptide substrat naturel SNAP25, par la BoNT/A, s'effectue au niveau de la liaison Q¹⁹⁷-R¹⁹⁸.

De la même manière que cela avait été effectué dans le cas du dosage de la BoNT/B avec un fragment de synaptobrévine , les inventeurs ont introduit de part et d'autre du site de clivage du SNAP25 par la toxine botulique de type A, un résidu Pya en position 195, 196 ou 197, et un résidu Nop en position 199, 200, ou 201 de ces différents fragments de SNAP25.

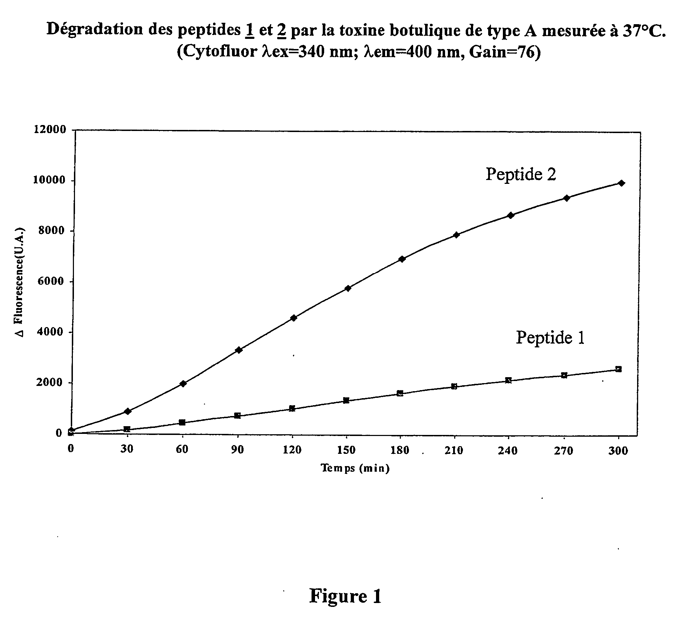

De façon inattendue, ces modifications ont conduit à la formation de peptides qui ne sont pas ou peu substrats de la BoNT/A. Ainsi, seul le peptide Pya¹⁹⁷Nop²⁰⁰(187-203)SNAP25 est faiblement clivé par la toxine BoNT/A à une concentration élevée (200 ng/ml), avec seulement un doublement de la fluorescence de base en environ 1h (Fig. 1).

Ce résultat suggère donc des différences importantes dans la structure du site actif des deux toxines botuliques A et B ainsi que dans leur mode d'action.

En revanche, de façon surprenante, le remplacement de la séquence Pya-(Z)-Nop par la séquence inverse Nop-(Z)-Pya, avec Z représentant un enchaînement d'aminoacides choisi de manière à ce que cette séquence inverse puisse être clivée par la BoNT/A, a permis d'obtenir d'excellents substrats de BoNT/A, dont la sensibilité est jusqu'à 100 fois supérieure à celle des substrats clivés par la BoNT/A et contenant la séquence Pya-(Z)-Nop.

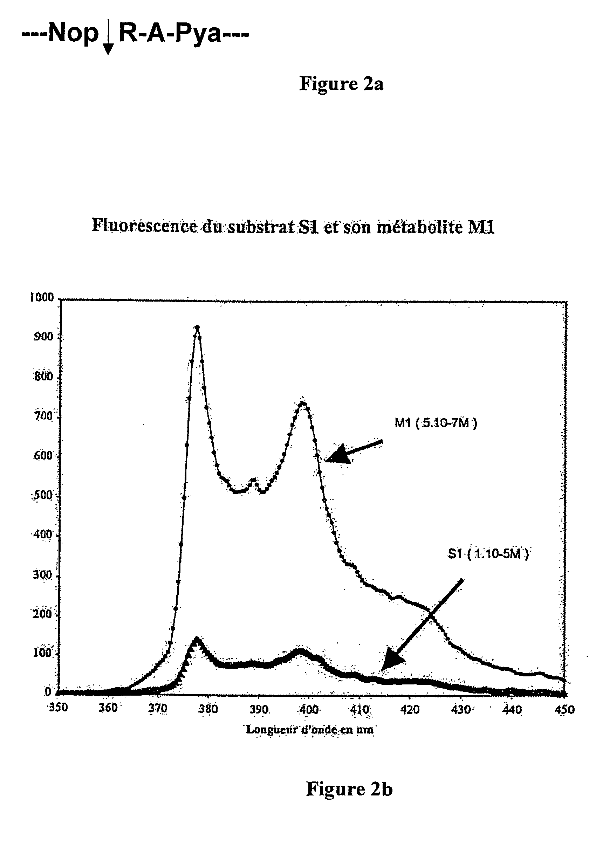

La présente invention repose donc sur le fait qu'il est possible de détecter une fluorescence intense provoquée par la séparation des résidus Nop et Pya intégrés dans un substrat de la BoNT/A lors du clivage enzymatique par cette neurotoxine d'une des liaisons situées entre ces résidus, à condition que le résidu Nop se retrouve dans le métabolite N-terminal et que le résidu Pya se retrouve dans le métabolite C-terminal. La figure 2a illustre ce phénomène.

Ainsi, la présente invention a principalement pour but de fournir un substrat peptidique reconnu par BoNT/A, utilisable dans le cadre de la mise en oeuvre de procédés pour détecter, identifier et/ou doser la toxine botulique de type A à des concentrations très faibles (de l'ordre de 20 pg), ou des inhibiteurs et/ou activateurs de l'activité métallopeptidasique de ladite toxine, et dans ce dernier cas pour quantifier leur puissance.

Un premier aspect de l'invention concerne donc un substrat peptidique, reconnu sélectivement par la toxine BoNT/A, caractérisé en ce qu'il comprend dans sa structure peptidique un fragment Nop-(Z)-Pya dans lequel Z représente un enchaînement d'aminoacides, ledit fragment étant clivé par la dite toxine.

Avantageusement Z représente de 2 à 4 aminoacides choisis de telle manière que le fragment Nop-(Z)-Pya susmentionné soit clivé par la BoNT/A.

Avantageusement, afin de préserver la spécificité et l'efficacité de la BoNT/A, les aminoacides des substrats de l'invention sont choisis parmi ceux de la séquence naturelle du SNAP25.

Avantageusement encore, Z contient les résidus arginine R en position 198 et A en position 199 de SNAP25 (Fig. 2a).

De préférence, le fragment Nop-(Z)-Pya des substrats de l'invention est choisi parmi les suivants :
1) Nop-R-A-Pya (SEQ ID NO: 3)
2) Nop-R-A-T-Pya (SEQ ID NO: 4)
3) Nop-R-A-T-K-Pya (SEQ ID NO: 5)

Selon un mode de réalisation préféré de l'invention, le reste (Z) comprend 2 aminoacides. De préférence, la séquence Nop-(Z)-Pya répond à la définition : Nop-R-A-Pya (SEQ ID NO : 3).

A ce titre, l'invention a plus particulièrement pour objet un substrat peptidique, reconnu par la toxine BoNT/A, caractérisé en ce qu'il comprend ou est constitué par la séquence suivante :
(X)ₘ-S-N-K-T-R-I-D-Xaa-A-N-Nop-R-A-Pya-K-Nle-(L)ₙ(GSG)ₚ (SEQ ID NO: 6)
dans laquelle :
- X représente un aminoacide, ou un enchaînement de 2 à 52 aminoacides,
- m, n et p, indépendamment l'un de l'autre représentent 0 ou 1,
- Xaa représente E ou Q,
à savoir tout substrat tel que défini ci-dessus comprenant ou constitué par la séquence (187-206)SNAP 25 dans laquelle :
- le résidu Q en position 197 est remplacé par un résidu Nop, et le résidu T en position 200 est remplacé par un résidu Pya ; le clivage par la BoNT/A d'une liaison peptidique située entre ces deux amino acides Nop et Pya conduit à l'exaltation de fluorescence sur laquelle repose l'invention,
- le résidu M en position 202 est remplacé par un résidu isostère tel que Nle,
- et, le cas échéant :
   le résidu E en position 194 est remplacé par un résidu Q,
   . et/ou le résidu L en position 203 est supprimé,
   . et/ou les résidus GSG aux positions 204 à 206 sont supprimés.

Avantageusement, les résidus N-terminal et C-terminal des substrats décrits ci-dessus et ci-après de l'invention sont protégés, notamment par acétylation et amidification respectivement, afin d'éviter toute dégradation non spécifique par des aminopeptidases et/ou des carboxypeptidases (par exemple lors de l'utilisation de toxines incomplètement purifiées).

L'invention a plus particulièrement pour objet un substrat tel que défini ci-dessus, caractérisé en ce que lorsque m représente 1, X représente :
- un résidu arginine ou un résidu cystéine dont la fonction thiol est le cas échéant immobilisée sur un support solide,
- ou un enchaînement de 2 à 52 aminoacides, correspondant à un fragment peptidique de la protéine SNAP25 (SEQ ID NO : 1) dont l'aminoacide C-terminal correspond à l'aminoacide situé en position 186 de la séquence de la protéine SNAP 25, et l'aminoacide N-terminal correspond à l'un des aminoacides situés en position 135 à 185 de la séquence de la protéine SNAP 25, l'un ou plusieurs des aminoacides susmentionnés étant le cas échéant remplacés par un aminoacide choisi parmi :A, S, E, F, L, Nle.

L'invention concerne plus particulièrement un substrat peptidique, reconnu par la toxine BoNT/A, caractérisé par la séquence suivante:
Ac-(X)ₘ-S-N-K-T-R-I-D-Xaa1-A-N-Nop-R-A-Pya-K-NIe-(L)ₙ-NH₂ (SEQ ID NO : 7)
dans laquelle :
- n= 0 ou 1,
- m = 0 ou 1,
- X = D ou C,
- Xaal représente E ou Q,
- et lorsque m = 1 et X = C, la fonction thiol du résidu cystéine est le cas échéant immobilisée sur un support solide, par exemple à l'aide d'un reste maleimide.

L'invention a plus particulièrement pour objet les substrats tels que définis ci-dessus comprenant la séquence (187-203)SNAP 25 susmentionnée dans laquelle :
- le résidu Q en position 197 est remplacé par un résidu Nop, et le résidu T en position 200 est remplacé par un résidu Pya,
- le résidu M en position 202 est remplacé par un résidu Nle,
- le résidu N-terminal S en position 187 est acétylé, ou protégé par un reste acétylcystéine,
- et le résidu C-terminal L en position 203 est amidifié,
à savoir les séquences S1 et S2 suivantes :
S1 : Ac-S-N-K-T-R-I-D-E-A-N-Nop-R-A-Pya-K-Nle-L-NH₂ (SEQ ID NO : 8)
S2 : Ac-C-S-N-K-T-R-I-D-E-A-N-Nop-R-A-Pya-K-Nle-L-NH₂ (SEQ ID NO : 9)

L'invention a plus particulièrement pour objet des substrats tels que définis ci-dessus, correspondant à une variante des séquences S1 et S2 dans laquelle le résidu E194 est remplacé par un résidu glutamine Q, à savoir les séquences S3 et S4 suivantes:
S3 : Ac-S-N-K-T-R-I-D-Q-A-N-Nop-R-A-Pya-K-Nle-L-NH₂ (SEQ ID NO : 10)
S4 : Ac-C-S-N-K-T-R-I-D-Q-A-N-Nop-R-A-Pya-K-Nle-L-NH₂ (SEQ ID NO : 11)

L'invention a plus particulièrement pour objet des substrats tels que définis ci-dessus, correspondant à une variante des séquences S3 et S4 dans laquelle la leucine N-terminale en position 203 est supprimée, à savoir les séquences S5 et S6 suivantes:
S5 : Ac-S-N-K-T-R-I-D-Q-A-N-Nop-R-A-Pya-K-Nle-NH₂ (SEQ ID NO : 12)
S6 : Ac-C-S-N-K-T-R-I-D-Q-A-N-Nop-R-A-Pya-K-Nle-NH₂ (SEQ ID NO: 13)

L'invention a également pour objet un substrat tel que défini ci-dessus, caractérisé en ce qu'il comprend ou est constitué par la séquence SEQ ID NO : 6 susmentionnée dans laquelle Xaa, n, et p sont tels que définis ci-dessus, m=1, et X représente un enchaînement d'aminoacides correspondant à la séquence KAD de SNAP 25 (184-186), ou sa séquence dérivée KSD.

A ce titre, l'invention a plus particulièrement pour objet un substrat tel que défini ci-dessus, caractérisé en ce qu'il comprend ou est constitué par la séquence S7 (SEQ ID NO : 14) suivante :
S7: Ac K S D S N K T R I D E A N Nop R A Pya K Nle L G S G NH₂ (SEQ ID NO : 14)

La vitesse de la réaction enzymatique de la BoNT/A sur ses substrats est extrêmement tributaire de la longueur du substrat (V.V.Vaidyanathan et al. J.Neuro.Chem.,1999,72,327-337) probablement à cause de la structure de la sous-unité enzymatique de la BoNT/A (Segelke et al. Proc. Natl Acad. Sci USA ,101,6888-6893,2004) et d'un mécanisme de type allostérique (Breidenbach et al. Nature, 432,925-929, 2004), avec existence d'exosites, similaire à celui démontré par les inventeurs pour la toxine tétanique (Cornille et al. J.Biol.Chem.,272,3459-3464,1997).

Ces résultats démontrent que la partie indispensable du SNAP 25 est constituée par le fragment C-terminal SNAP 25 (187-203), mais que l'affinité maximale pour la toxine est donnée par la séquence précédant le résidu 187 du SNAP 25 pourvu qu'elle contienne les séquences se fixant aux exosites, en particulier les séquences 156-186 ou 146-186.

La présente invention a donc pour objet de proposer des substrats préférés de toxine botulique de type A capables d'être hydrolysés très rapidement, ceci permettant de détecter cette toxine à des concentrations égales ou inférieures à la dose létale chez l'homme (de l'ordre de 1 µg/70 kg). En effet, les applications de l'invention sont particulièrement dévolues à la détection et éventuellement la quantification de la BoNT/A dans l'eau, les différents produits alimentaires et l'air qui seraient infectés, notamment en cas de bioterrorisme. La seconde importante application est la détermination très précise des concentrations de BoNT/A dans des produits pharmaceutiques utilisés pour différentes applications médicales et esthétiques (Schantz et Johnson Microbiol. Rev.,1992, 56, 80-99). Dans ce dernier cas, la technique revendiquée permet effectivement de doser quantitativement une concentration de l'ordre de 10 DL 50 souris c'est-à-dire environ 20 pg. Des concentrations aussi faibles doivent, obligatoirement, être déterminées dans les spécialités pharmaceutiques de manière à remplacer toutes les méthodes in vitro et in vivo existant à ce jour.

A ce titre, l'invention a plus particulièrement pour objet un substrat peptidique reconnu sélectivement par la toxine botulique de type A, BoNT/A, caractérisé en ce qu'il comprend ou est constitué par la séquence SEQ ID NO : 6 susmentionnée dans laquelle Xaa, n, et p sont tels que définis ci-dessus, m=1, et X représente un enchaînement d'au moins 30 aminoacides.

L'invention concerne plus particulièrement un substrat tel que défini ci-dessus, caractérisé en ce que X représente un enchaînement d'au moins 30 aminoacides, correspondant à un fragment peptidique de la protéine SNAP25 dont l'aminoacide C-terminal correspond à l'aminoacide situé en position 186 de la séquence de la protéine SNAP 25, et l'aminoacide N-terminal correspond à l'un des aminoacides situés en position 135 à 156 de la séquence de la protéine SNAP 25 (SEQ ID NO : 1), l'un ou plusieurs des aminoacides susmentionnés étant le cas échéant remplacés par un aminoacide choisi parmi :A, S, E, F, L, Nle.

L'invention a plus particulièrement pour objet un substrat tel que défini ci-dessus, caractérisé en ce que X représente un enchaînement d'aminoacides correspondant :
- à la séquence SNAP 25 (135-186), ou ses séquences dérivées suivantes :
- à la séquence SNAP 25 (141-186), ou ses séquences dérivées suivantes :
- à la séquence SNAP 25 (146-186), ou ses séquences dérivées suivantes :
- ou à la séquence SNAP 25 (156-186), ou ses séquences dérivées suivantes :
dans lesquelles Xaa1, Xaa2, Xaa3, et Xaa4, indépendamment les uns des autres, représentent M ou Nle.

L'invention concerne plus particulièrement un substrat tel que défini ci-dessus, caractérisé en ce qu'il comprend ou est constitué par l'une des séquences suivantes :
- S8 : SEQ ID NO : 19 :
- S9: SEQ ID NO : 20 :
- S10 : SEQ ID NO : 21 :

Un substrat tel que défini ci-dessus particulièrement préféré selon l'invention est celui caractérisé en ce qu'il comprend ou est constitué par la séquence S9 : SEQ ID NO : 20 suivante :

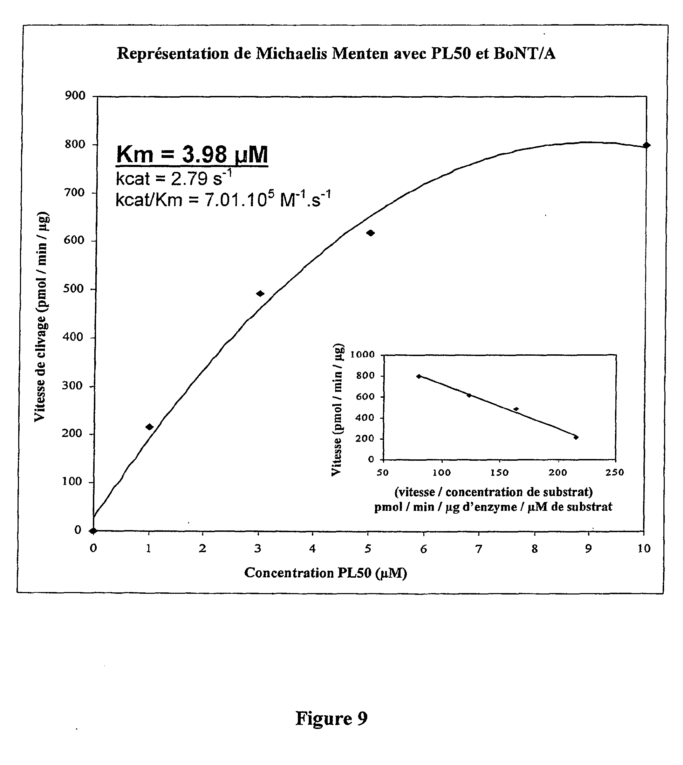

Les paramètres (Km, kcat, Vmax) de l'hydrolyse de S9 (voir figure 9) s'avèrent meilleurs que ceux rapportés pour le SNAP 25 lui-même et d'autres substrats rapportés à ce jour.

L'invention concerne également les fragments, ou métabolites, non fluorescents et fluorescents issus du clivage (figure 1a) des substrats peptidiques de l'invention par la BoNT/A, les métabolites non fluorescents étant caractérisés en ce qu'ils comprennent le résidu Nop, et les métabolites fluorescents étant caractérisés en ce qu'ils comprennent le résidu Pya.

L'invention a plus particulièrement pour objet les métabolites fluorescents susmentionnés issus du clivage des substrats peptidiques de l'invention par la BoNT/A au niveau de la liaison Nop¹⁹⁷-¹⁹⁸R, à savoir les métabolites de formule:
R-A-Pya-K-Nle-(L)ₙ(GSG)ₚ-NH₂ (SEQ ID NO : 22)
dans laquelle n et p sont tels que définis ci-dessus,
et plus particulièrement :
- le métabolite M1 fluorescent suivant, issu notamment du clivage des substrats S1, S2, S3, S4, S8, S9, et S10 par la BoNT/A au niveau de la liaison Nop¹⁹⁷-¹⁹⁸R,
   - M1 :: R-A-Pya-K-Nle-L-NH₂ (SEQ ID NO : 23)
- le métabolite M2 fluorescent suivant, issu notamment du clivage des substrats S5, et S6 par la BoNT/A au niveau de la liaison Nop¹⁹⁷-¹⁹⁸R,
   - M2 :: R-A-Pya-K-Nle-NH₂ (SEQ ID NO : 24).
- le métabolite M3 fluorescent suivant, issu notamment du clivage du substrat S7 par la BoNT/A au niveau de la liaison Nop¹⁹⁷-¹⁹⁸R,
   - M3 :: R-A-Pya-K-Nle-L-G-S-G-NH₂ (SEQ ID NO : 25).

Les substrats conformes à la présente invention sont intéressants à plusieurs titres :
- ils permettent de détecter rapidement au sein d'un liquide ou d'une substance la présence éventuelle de la toxine botulique de type A, à des concentrations inférieures à la dose létale chez l'homme,
- ils présentent une très haute sélectivité vis à vis des autres toxines botuliques, et autres peptidases,
- ils rendent possible le criblage à haut débit d'inhibiteurs potentiels de la toxine botulique de type A,
- ils donnent naissance à seulement deux métabolites du fait de la coupure sélective de la liaison Nop-R par la BoNT/A.

En conséquence, la présente invention concerne l'utilisation d'un substrat tel que défini précédemment pour la mise en oeuvre de procédés de détection, d'identification et/ou de dosage de la BoNT/A ou d'un composé capable d'inhiber ou d'activer la BoNT/A, et pour la mise en oeuvre de méthodes de diagnostic in vitro de la présence de la BoNT/A dans un échantillon issu de milieux divers tels que l'eau, l'air, le lait, les boissons, les aliments, et les milieux biologiques tels que le sang, l'urine, et le liquide céphalorachidien, ainsi que pour la détermination précise des concentrations de la BoNT/A au cours de la fabrication, de la purification, et du conditionnement de compositions pharmaceutiques aux fins d'usage thérapeutique chez l'homme ou l'animal (notamment dans le cadre de pathologies neurologiques), ou de compositions cosmétiques.

Selon une variante préférée de l'invention, les substrats sont utilisés pour rechercher des inhibiteurs de la BoNT/A et quantifier leur pouvoir inibiteur.

La présente invention a également pour objet un procédé pour détecter, identifier et/ou doser un composé capable d'inhiber la BoNT/A caractérisé en ce qu'il comprend :
- la mise en présence en solution d'un substrat conforme à l'invention avec ladite BoNT/A ou sa chaîne légère (telles que décrites notamment dans Cai and Singh, Biochemistry (2001) 40, 4693-4702), et au moins un composé susceptible d'inhiber la BoNT/A;
- la mesure de la fluorescence, émise par les métabolites fluorescents susmentionnés formés lors de l'étape précédente d'hydrolyse du substrat, en présence et/ou en l'absence dudit composé, une diminution de fluorescence entre la mesure effectuée en présence de ce composé et celle effectuée en l'absence de ce composé, indiquant alors que le composé testé est inhibiteur de BoNT/A.

L'addition de doses croissantes d'un composé capable d'inhiber l'activité enzymatique de la BoNT/A vis-à-vis d'un substrat conforme à la présente invention, se traduira par une diminution de l'intensité de la fluorescence due à la diminution de la quantité de métabolite formé par la BoNT/A. La mesure de cette intensité rapportée à une courbe étalon établie par mélange du substrat et du métabolite fluorescent formé, permet donc soit d'évaluer le pouvoir inhibiteur en pourcentage d'inhibition à une concentration donnée fixe du composé inhibiteur, soit de déterminer précisément son IC50 puis son Ki à l'aide du Km du substrat et de plusieurs concentrations du produit inhibiteur.

Un composé auquel on applique le test défini ci-dessus pour déterminer son activité inhibitrice vis à vis de la BoNT/A peut être de nature diverse, sans limitation et peut se présenter sous une forme isolée, être connu ou inconnu et/ou être présent dans une banque de composés, un extrait biologique.

L'extrême sensibilité du dosage permet d'opérer dans des volumes très faibles (50 ou 100 µl) avec des concentrations de l'ordre de 5 à 50 µM du substrat choisi (notamment S5, S8 et S9). Le test est donc utilisable en plaques à 96 puits ou plus, permettant sa robotisation avec détermination automatique des Ki lorsque le fluorimètre de lecture est relié à un ordinateur possédant un logiciel commercial approprié.

La présente invention propose donc un test d'identification d'inhibiteurs de la BoNT/A ainsi que la détermination de leur pouvoirs inhibiteurs par un essai fluorimétrique très rapide, reproductible et permettant des tests robotisables et très nombreux pouvant s'adapter à une sélection à haut débit de molécules inhibitrices.

La présente invention propose également la production de produits industriels nouveaux, éventuellement utilisables sous forme de kits, notamment comprenant des plaques de 96, 192 et 384 puits prêts à l'emploi, et contenant un substrat tel que défini ci-dessus selon l'invention et les réactifs nécessaires à la détermination des pouvoirs inhibiteurs ou activateurs de la BoNT/A. La présente invention propose en outre des kits comprenant des comprimés contenant ensemble un des substrats de l'invention (en particulier S9) et les réactifs nécessaires au développement de la réaction enzymatique et donc à l'apparition d'une fluorescence intense en cas d'addition d'eau ou d'une substance contenant la BoNT/A. Dans ce cas, avantageusement les kits de l'invention comprennent également un fluorimètre, dont, de préférence, la longueur d'onde d'émission est comprise entre 360 et 420 nm, et la longueur d'onde d'excitation est comprise entre 320 et 350 nm ainsi que les réactifs EDTA et/ou gangliosides séparés ou associés et toutes substances capables d'inhiber les enzymes autres que la BoNT/A sans affecter l'activité de cette dernière.

Un autre aspect de l'invention concerne une méthode de diagnostic *in vitro* de la présence de la BoNT/A dans un échantillon tel que défini ci-dessus, ou de mesure des concentrations de la BoNT/A au cours des étapes de fabrication, purification, et conditionnement de compositions pharmaceutiques ou cosmétiques dans un échantillon prélevé lors de ces étapes.

L'invention concerne également une méthode de diagnostic *in vitro* de la présence de la BoNT/A dans l'organisme humain ou animal, mettant en oeuvre un substrat conforme à l'invention.

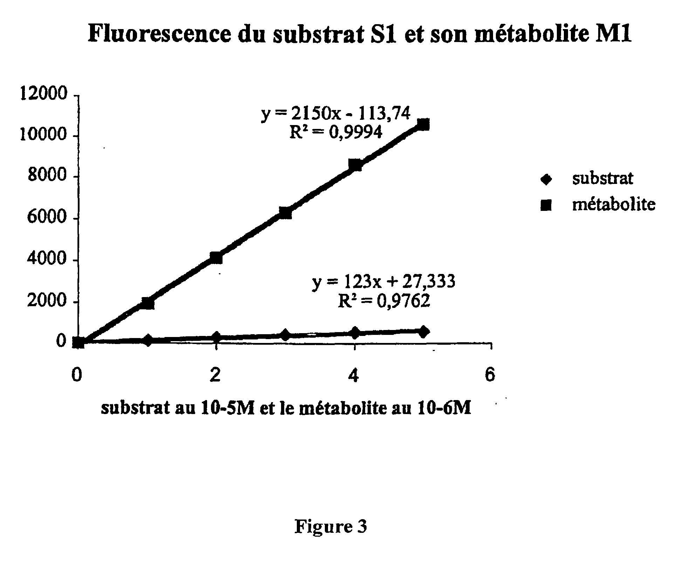

De telles méthodes de diagnostic ou de mesure comprennent :
- une étape de mise en présence en solution d'un substrat conforme à l'invention avec un échantillon tel que défini ci-dessus (par exemple des prélèvements effectués sur des aliments, des liquides, ou des prélèvements sanguins humains, éventuellement contaminés par la BoNT/A);
- la mesure de la fluorescence émise par les éventuels métabolites fluorescents susmentionnés formés lors de l'étape précédente, témoignant de la présence de BoNT/A dans ledit échantillon, qui peut être quantifiée à l'aide de courbes étalons appropriées (voir figures annexées),
- le cas échéant et dans le but d'écarter des réponses faussement positives, l'élimination de la fluorescence émise lors de l'étape susmentionnée par addition d'un chélatant du zinc, comme par exemple, et de manière non limitative, l'éthylène diamine tétracétate EDTA qui inhibe l'activité métalloprotéasique de la BoNT/A (figure 3), ou par addition de gangliosides, qui bloquent cette dernière par formation d'un complexe inactif.

Les figures et exemples annexées sont présentés à titre illustratif et non limitatif de la présente invention.

### Figures :

- Figure 1 : Différence d'exaltation de fluorescence lors de la coupure par la BoNT/A du substrat 1 (peptide 1), Ac-S-N-K-T-R-I-D-E-AN-***Pya***-R-A-***Nop***-K-Nle-L-NH₂ (■, graphique inférieur) et 2 (peptide 2); Ac-S-N-K-T-R-I-D-E-A-N-***Nop***-R-A-***Pya***-K-Nle-L-NH₂ (◆, courbe supérieure). En abscisse est représenté le temps en minutes, et en ordonnée la fluorescence (U.A.).
- Figure 2 : a) Schéma du clivage des substrats selon l'invention par la BoNT/A.
   b) Comparaison de l'émission de fluorescence du substrat S1 (10⁻⁵ M) et son métabolite M1 (à 5 10⁻⁷M correspondant à 5% de dégradation). Excitation à 343 nm. En abscisse est indiquée la longueur d'onde en nm et en ordonnée l'intensité émise.
- Figure 3 : Courbe d'étalonnage de la fluorescence pour la détermination des % de substrat dégradé au cours d'un dosage.

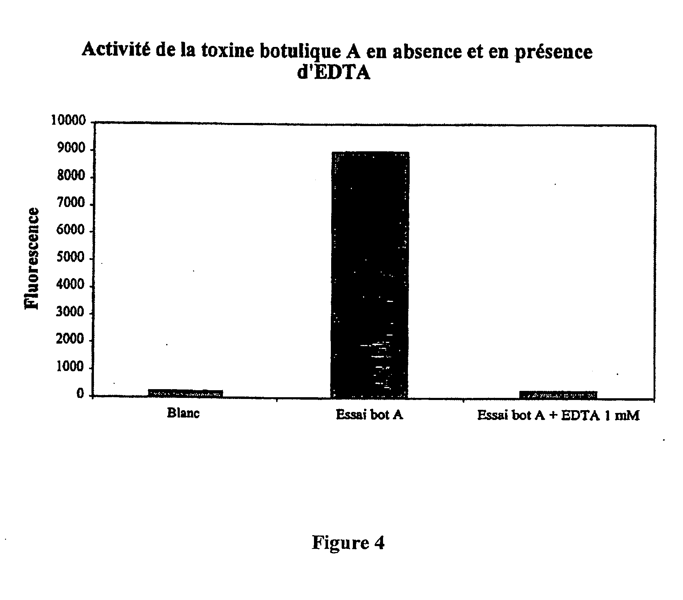

- Figure 4 : Fluorescence émise par la dégradation du substrat S1 (50 µM) en 1 h par la BoNT/A et inhibition de la fluorescence (donc de l'activité enzymatique par l'EDTA).
- Figure 5 : Essai fluorimétrique de quantification de la BoNT/A avec le substrat S9. Les concentrations sont exprimées en LD 50 souris, soit 1 DL 50 = 2 pg. En abscisse est indiqué le temps en minutes, et en ordonnée la fluorescence (U.A.).
- Figure 6 : Relation linéaire entre exaltation de fluorescence (en ordonnée, U.A.) et concentration de BoNT/A (en abscisse, en DL50) à un temps déterminé avec le substrat S9.

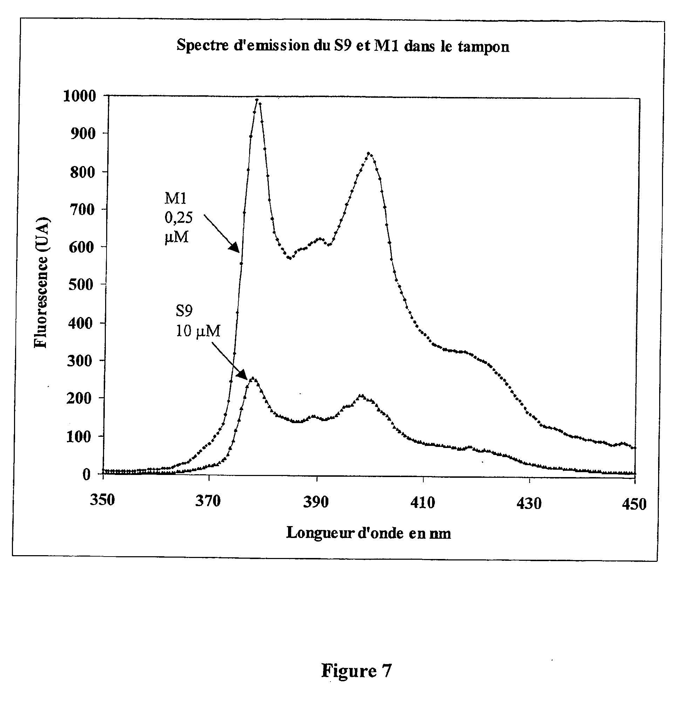

- Figure 7 : Emission de fluorescence du substrat S9 et son métabolite M1 correspondant à 2,5% de dégradation ; excitation à 343 nm. En abscisse est indiquée la longueur d'onde en nm, et en ordonnée la fluorescence en U.A.

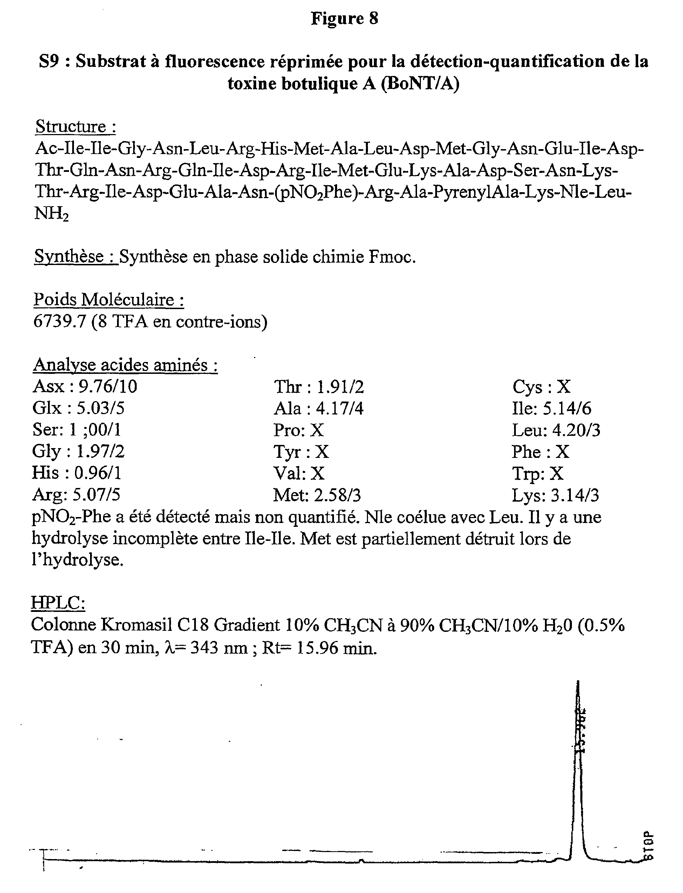

- Figure 8 : Paramètres analytiques de S9.
- Figure 9 : Paramètres cinétiques de la dégradation de S9 par la BoNT/A. Le Km et les constantes catalytiques se révèlent plus favorables que pour le SNAP 25. En abscisse est indiquée la concentration PL50 en µM et en ordonnée la vitesse de clivage (en pmol / min / µg). Insert : rapport vitesse / concentration du substrat en pmol/min/µg d'enzyme /µM de substrat en abscisse et vitesse en pmol/min/µg en ordonnée.

### Exemples

### 1) Synthèse des peptides

Les composés revendiqués peuvent être obtenus par les méthodes usuelles de la synthèse en phase solide selon la méthode de Merrifield sur un synthétiseur automatique comme par exemple l'appareil 431A de Applied Biosystems. La chimie utilisée correspond à la technologie Fmoc. La déprotection des chaînes latérales et le clivage de la peptidylrésine sont effectués par l'acide trifluoroacétique tel que décrit par E. Atherton et R.C. Sheppard (1989) dans "Solid phase peptides synthesis: a practical approach, IRL Press,Oxford " .Les couplages sont effectués selon les techniques conventionnelles à l'aide d'agents de couplage comme HATU, Bop, PyBrop et de préférence en utilisant le dicyclohexylcarbodiimide (DCC) en présence d'hydroxybenzotriazole (HOBT). Des détails sur les techniques utilisées peuvent être trouvées dans les publications rapportant la synthèse de très grands peptides effectués par les inventeurs (Cornille F. et al., J. Pept. Res., 1999, 54, 427-435 ; Comille F. et al., Nucleic Ac. Res., 1998, 26, 2143-2149 ; Cornille F. et al., Int. J. Pept. Rot. Res., 1990, 36, 551-558 ; de Rocquigny H. et al., Proc. Natl. Acad. Sci., 1992, 89, 6472-6476). La L-pyrenylalanine est obtenue selon un procédé de synthèse asymétrique décrit dans la publication Soleilhac et al. (Anal. Biochem., 1996, 241, 120-127).
Les peptides sont purifiés par HPLC semi-préparative. La pureté des peptides est estimée supérieure à 95% par HPLC en phase inverse et leur identification est obtenue par spectrométrie de masse.
- Substrat S1 :: Ac-S-N-K-T-R-I-D-E-A-N-Nop-R-A-Pya-K-Nle-L-NH₂ (SEQ ID NO: 8):
M (théo) 2233,53 ; MH⁺ (exp) 2234,20
- Substrat S2 :: Ac-C-S-N-K-T-R-I-D-E-A-N-Nop-R-A-Pya-R-A-Pya-K-Nle-L-NH₂ (SEQ ID NO : 9) :
M (théo) 2336,67 ; MH⁺ (exp) 2337,98
- Substrat S3 :: Ac-S-N-K-T-R-I-D-Q-A-N-Nop-R-A-Pya-K-Nle-L-NH₂ (SEQ ID NO : 10) :
M (théo) 2232,55 ; MH⁺ (exp) 2233,78
- Substrat S4 :: Ac-C-S-N-K-T-R-I-D-Q-A-N-Nop-R-A-Pya-K-Nle-L-NH₂ (SEQ ID NO : 11) :
M (théo) 2335,69 ; MH⁺ (exp) 2336,48
- Substrat S5 :: Ac-S-N-K-T-R-I-D-Q-A-N-Nop-R-A-Pya-K-Nle-NH₂ (SEQ ID NO : 12) :
M (théo) 2119,39 ; MH⁺ (exp) 2120,54
- Substrat S6 :: Ac-C-S-N-K-T-R-I-D-Q-A-N-Nop-R-A-Pya-K-Nle-NH₂ (SEQ ID NO : 13) :
M (théo) 2222,53 ; MH⁺ (exp) 2223,80
- Métabolite M1 :: R-A-Pya-K-Nle-L-NH₂ (SEQ ID NO : 23)
M (théo) 867,12 ; MH⁺ (exp) 868,24
- Métabolite M2 :: R-A-Pya-K-Nle-NH₂ (SEQ ID NO : 24).
M(théo) 756,96 ; MH⁺ (exp) 758,02
- Substrat S7:: Ac K S D S N K T R I D E A N Nop R A Pya K Nle L G S G NH₂ (SEQ ID NO : 14) : M(théo): 2764.81; M(exp): 2764.0
- Substrat S9 :: Ac I I G N L R H M A L D M G N E I D T Q N R Q I D R I M E K A D S N K T R I D E A N Nop R A Pya K Nle-L NH₂ (SEQ ID NO : 22) : M(théo): 5827.7; M (exp): 5826.9
- Substrat S10 :: Ac I I G N L R H Nle A L D Nle G N E 1 D T Q N R Q I D R I Nle E K A D S N K T R I D E A N Nop R A Pya K Nle L NH₂ (SEQ ID NO: 23) : M (théo): 5773.58; M (exp): 5773.2

### 2) Caractéristiques physico-chimiques du substrat S9

| Analyse acides aminés : | | |
|---|---|---|
| Asx : 9.76/10 | Thr : 1.91/2 | Cys : X |
| Glx : 5.03/5 | Ala : 4.17/4 | Ile: 5.14/6 |
| Ser: 1 ;00/1 | Pro: X | Leu: 4.20/3 |
| Gly: 1.97/2 | Tyr : X | Phe : X |
| His : 0.96/1 | Val: X | Trp: X |
| Arg: 5.07/5 | Met: 2.58/3 | Lys: 3.14/3 |

| | | |
|---|---|---|
| pNO₂-Phe a été détecté mais non quantifié. Nle coélue avec Leu. Il y a une hydrolyse incomplète entre Ile-Ile. Met est partiellement détruit lors de l'hydrolyse (voir également HPLC de S9, figure 8). | | |

### 3) Essai fluorimétrique de détection de la BoNT/A

On incube le substrat (S1 par exemple) 50µM pendant 1h avec une quantité inconnue de BoNT/A double chaîne à 37°C dans 100mL de tampon Hépes 20mM, pH 7,4, contenant de la BSA (1mg/mL), du ZnSO4 (0,3mM) et du DTT (5mM). On arrête l'hydrolyse enzymatique en plaçant l'échantillon à 4°C, ou en ajoutant 5 µL d'HCl 2N, et on lit directement la fluorescence émise au fluorimètre (λex 343 nm, λem 377 nm).

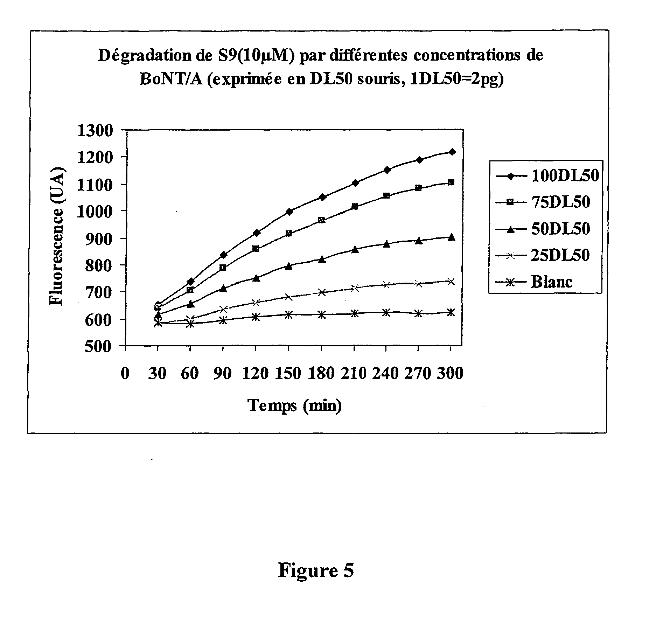

L'intensité de fluorescence mesurée est rapportée à une courbe étalon pour établir la quantité de BoNT/A présente dans l'échantillon. Les expériences effectuées avec plusieurs quantités de BoNT/A montrent que la précision atteint ± 10% de la quantité de neurotoxine dans la gamme 0,2 ng/ml à 15 ng/ml (voir figure 5).

### 4) Essai fluorimétrique de détection de la BoNTA avec le substrat S9 (figure 5)

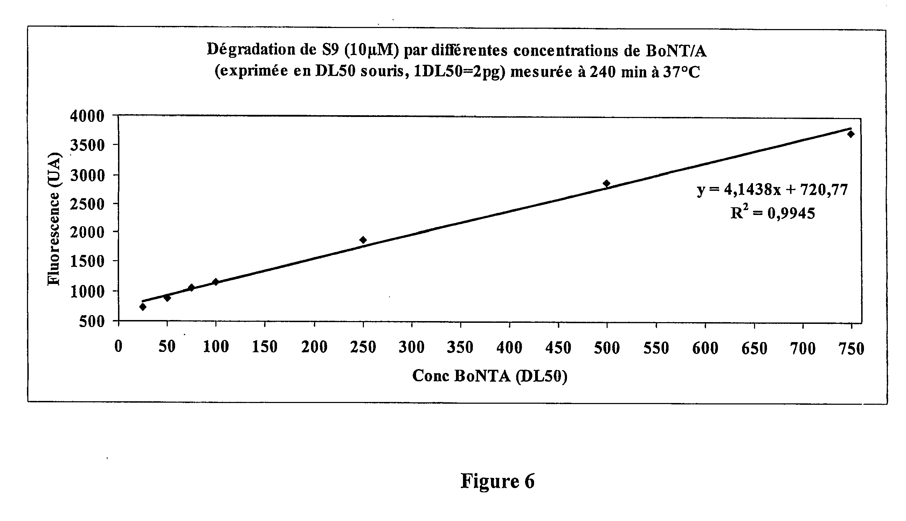

On incube le substrat S9 10µM en fonction du temps avec différentes concentrations de BoNT/A double chaîne (1DL50 souris = 2pg) à 37°C dans 100µL de tampon Hépes 20mM, pH 7,4, contenant de la BSA (1mg/mL), du ZnSO₄ (0,3mM) et du DTT (5mM). On lit directement la fluorescence émise avec un appareil cytofluor (λex 340 nm, λem 400 nm). Son augmentation par rapport au contrôle est directement reliée à la concentration de BoNTA et ce à chaque temps. Ceci permet donc de déterminer dans un échantillon de concentration en BoNTA inconnue la quantité de toxine (voir figure 6). Avec S9 une concentration de BoNT/A de 0.2 ng/ml peut être quantifiée.
Ces expériences peuvent être réalisées avec différents types de fluorimètre et des volumes d'échantillon variables par exemple de 50µl à 2ml.

### 5) Détermination du site de clivage de S9 (10µM) par BoNTA (50ng/100µl).

L'analyse a été effectué en parallèle par LC-MS (Kromasil C18 CH₃CN/H₂₀(0.5 % TFA) gradient 10% CH3CN à 90% en 60 min; masse electrospray sur LCD Avantage Thermo Finnigan) et HPLC (Kromasil C18 CH₃CN/H₂₀(0.5 % TFA) gradient 10% CH3CN à 90% en 30 min.

Le métabolite fluorescent M1 est détecté avec un m/z=870.1 correspondant à M+H.

### Métabolite M1: R A Pya K Nle-L NH₂

Le métabolite non fluorescent M3 est détecté avec m/z= 4975.0 correspondant à (M+H)
Métabolite M4:Ac I I G N L R H M A L D M G N E I D T Q N R Q I D R I M E K A D S N K T R I D E A N Nop (SEQ ID NO : 26)

Il a également été vérifié par HPLC (co-injection avec les métabolites synthétisés) qu'il n'y a que deux métabolites formés.

### 6) Comparaison de la fluorescence induite par action de la toxine botulique de type A sur les substrats à fluorescence réprimés suivants (Figure 1)

Peptide 1: Ac-S-N-K-T-R-T-D-E-A-N-***Pya***-R-A-***Nop***-K-Nle-L-NH₂ (SEQ ID NO : 27)
Peptide 2: Ac-S-N-K-T-R-I-D-E-A-N-***Nop***-R-A-***Pya***-K-Nle-L-NH₂ (SEQ ID NO : 8)

L'expérience (voir figure 1) montre la très nette supériorité de l'arrangement Nop-Z-Pya par rapport à Pya-Z-Nop. Ce dernier donne une variation de fluorescence beaucoup trop faible pour répondre aux exigences du présent brevet (dosage précis de BoNT/A dans les préparations pharmaceutiques, détection de doses très faibles de BoNT/A susceptibles de générer des effets toxiques chez l'homme dans diverses préparations, en particulier l'eau.

## Revendications

1. Substrat peptidique reconnu sélectivement par la toxine botulique de type A, BoNT/A, **caractérisée en ce qu'**il comprend dans sa structure peptidique un fragment Nop-(Z)-Pya clivable par la dite toxine et dans lequel Z représente un enchaînement de 2 à 4 aminoacides.

2. Substrat selon la revendication 1, **caractérisé en ce que** le fragment Nop-(Z)-Pya est choisi parmi les suivants :
1) Nop-R-A-Pya (SEQ ID NO : 3)
2) Nop-R-A-T-Pya (SEQ ID NO : 4)
3) Nop-R-A-T-K-Pya (SEQ ID NO : 5)

3. Substrat selon l'une quelconque des revendications 1 et 2, **caractérisé en ce que** le fragment Nop-(Z)-Pya correspond à la séquence Nop-R-A-Pya (SEQ ID NO : 3).

4. Substrat peptidique selon l'une des revendications 1 à 3, reconnu sélectivement par la toxine botulique de type A, BoNT/A, **caractérisé en ce qu'**il comprend ou est constitué par la séquence suivante :
Ac-(X)ₘ-S-N-K-T-R-I-D-Xaa-A-N-Nop-R-A-Pya-K-Nle-(L)ₙ(GSG)ₚ-NH₂ (SEQ ID NO: 6)
dans laquelle :
- X représente un aminoacide, ou un enchaînement de 2 à 52 aminoacides,
- m, n et p, indépendamment l'un de l'autre représentent 0 ou 1,
- Xaa représente E ou Q.

5. Substrat selon l'une des revendications 1 à 4, **caractérisé en ce que** lorsque m représente 1, X représente :
- un résidu arginine ou un résidu cystéine dont la fonction thiol, est le cas échéant, immobilisée sur un support solide
- ou un enchaînement de 2 à 52 aminoacides, correspondant à un fragment peptidique de la protéine SNAP25 dont l'aminoacide C-terminal correspond à l'aminoacide situé en position 186 de la séquence de la protéine SNAP 25, et l'aminoacide N-terminal correspond à l'un des aminoacides situés en position 135 à 185 de la séquence de la protéine SNAP 25, l'un ou plusieurs des aminoacides susmentionnés étant le cas échéant remplacés par un aminoacide choisi parmi :A, S, E, F, L, Nle.

6. Substrat selon l'une des revendications 1 à 5, **caractérisé en ce qu'**il comprend ou est constitué par les séquences S1 à S6 suivantes :
S1 : Ac-S-N-K-T-R-I-D-E-A-N-Nop-R-A-Pya-K-Nle-L-NH₂ (SEQ ID NO : 8)
S2 : Ac-C-S-N-K-T-R-I-D-E-A-N-Nop-R-A-Pya-K-Nle-L-NH₂ (SEQ ID NO : 9)
S3 : Ac-S-N-K-T-R-I-D-Q-A-N-Nop-R-A-Pya-K-Nle-L-NH₂ (SEQ ID NO : 10)
S4 : Ac-C-S-N-K-T-R-I-D-Q-A-N-Nop-R-A-Pya-K-Nle-L-NH₂ (SEQ ID NO : 11)
S5: Ac-S-N-K-T-R-I-D-Q-A-N-Nop-R-A-Pya-K-Nle-NH₂ (SEQ ID NO : 12)
S6 : Ac-C-S-N-K-T-R-I-D-Q-A-N-Nop-R-A-Pya-K-Nle-NH₂ (SEQ ID NO : 13)

7. Substrat selon l'une des revendications 1 à 5, **caractérisé en ce que** X représente un enchaînement d'aminoacides correspondant à la séquence KAD de SNAP 25 (184-186), ou sa séquence dérivée KSD.

8. Substrat selon la revendication 5, **caractérisé en ce qu'**il comprend ou est constitué par la séquence S7 (SEQ ID NO : 14) suivante :
S7: Ac K S D S N K T R I D E A N Nop R A Pya K Nle L G S G NH₂ (SEQ ID NO : 14)

9. Substrat selon l'une des revendications 1 à 5, **caractérisé en ce que** X représente un enchaînement d'aminoacides d'au moins 30 aminoacides.

10. Substrat selon la revendication 9, **caractérisé en ce que** X représente un enchaînement d'aminoacides correspondant :
- à la séquence SNAP 25 (135-186), ou ses séquences dérivées suivantes :
- à la séquence SNAP 25 (141-186), ou ses séquences dérivées suivantes :
- à la séquence SNAP 25 (146-186), ou ses séquences dérivées suivantes :
- ou à la séquence SNAP 25 (156-186), ou ses séquences dérivées suivantes :
dans lesquelles Xaa1, Xaa2, Xaa3, et Xaa4, indépendamment les uns des autres, représentent M ou Nle.

11. Substrat selon les revendications 9 ou 10, **caractérisé en ce qu'**il comprend ou est constitué par l'une des séquences S8 à S10 suivantes:
- S8: SEQ ID NO : 19 :
- S9: SEQ ID NO : 20 :
- S10: SEQ ID NO : 21 :

12. Substrat selon l'une des revendications 9 à 11, **caractérisé en ce qu'**il comprend ou est constitué par la séquence S9 (SEQ ID NO : 20) suivante :
Ac I I G N L R H M A L D M G N E I D T Q N R Q I D R I M E K A D S N K T R I D E A N Nop R A Pya K Nle L NH₂

13. Métabolites non fluorescents et fluorescents issus du clivage des substrats peptidiques définis dans l'une des revendications 1 à 12, par la BoNT/A, les métabolites non fluorescents étant **caractérisés en ce qu'**ils comprennent le résidu Nop, et les métabolites fluorescents étant **caractérisés en ce qu'**ils comprennent le résidu Pya.

14. Métabolites fluorescents selon la revendication 13, issus du clivage des substrats peptidiques selon l'une quelconque des revendications 1 à 13 par la BoNT/A au niveau de la liaison Nop¹⁹⁷-¹⁹⁸R, à savoir les métabolites de formule :
R-A-Pya-K-Nle-(L)ₙ(GSG)_{P}-NH₂ (SEQ ID NO : 22)
dans laquelle n et p sont tels que définis dans la revendication 5.

15. Métabolites fluorescents selon la revendication 13 ou 14, suivants :
- le métabolite M1 fluorescent suivant, issu du clivage des substrats S1, S2, S3, S4, et S8 à S10 par la BoNT/A au niveau de la liaison Nop¹⁹⁷-¹⁹⁸R,
M1 : R-A-Pya-K-Nle-L-NH₂ (SEQ ID NO : 23),
- le métabolite M2 fluorescent suivant, issu du clivage des substrats S5 et S6 par la BoNT/A au niveau de la liaison Nop¹⁹⁷-¹⁹⁸R,
M2 : R-A-Pya-K-Nle-NH₂ (SEQ ID NO : 24),
- le métabolite M3 fluorescent suivant, issu du clivage du substrat S7 par la BoNT/A au niveau de la liaison Nop¹⁹⁷-¹⁹⁸R,
M3 : R-A-Pya-K-Nle-L-G-S-G-NH₂ (SEQ ID NO : 25).

16. Utilisation d'un substrat selon l'une des revendications 1 à 12, pour la mise en oeuvre de procédés de détection, d'identification et/ou de dosage de la BoNT/A ou d'un composé capable d'inhiber ou d'activer la BoNT/A, et pour la mise en oeuvre de méthodes de diagnostic *in vitro* de la présence de la BoNT/A dans un échantillon issu de milieux divers tels que l'eau, l'air, le lait, les boissons, les aliments, et les milieux biologiques tels que le sang, l'urine, et le liquide céphalorachidien, ainsi que pour la détermination précise des concentrations de la BoNT/A au cours de la fabrication, de la purification, et du conditionnement de compositions pharmaceutiques aux fins d'usage thérapeutique chez l'homme ou l'animal, ou de compositions cosmétiques.

17. Procédé pour détecter, identifier et/ou doser un composé capable d'inhiber la BoNT/A, **caractérisé en ce qu'**il comprend :
- la mise en présence en solution d'un substrat selon l'une des revendications 1 à 13 avec la BoNT/A et au moins un composé susceptible d'inhiber la BoNT/A
- la mesure de la fluorescence, émise par les métabolites fluorescents selon l'une des revendications 13 à 15, formés lors de l'étape précédente, en présence et/ou en l'absence dudit composé, une diminution de fluorescence entre la mesure effectuée en présence de ce composé et celle effectuée en l'absence de ce composé, indiquant alors que le composé testé est inhibiteur de BoNT/A.

18. Méthode de diagnostic *in vitro* de la présence de la BoNT/A dans un échantillon tel que défini dans la revendication 16, ou de mesure des concentrations de la BoNT/A au cours des étapes de fabrication, purification, et conditionnement de compositions pharmaceutiques ou cosmétique dans un échantillon prélevé lors de ces étapes, comprenant :
- une étape de mise en présence en solution d'un substrat selon l'une des revendications 1 à 12 avec ledit échantillon;
- la mesure de la fluorescence émise par les éventuels métabolites fluorescents selon l'une des revendications 13 à 15 formés lors de l'étape précédente, témoignant de la présence de BoNT/A dans ledit échantillon, qui peut être quantifiée à l'aide de courbes étalons appropriées,
- le cas échéant et dans le but d'écarter des réponses faussement positives, l'élimination de la fluorescence émise lors de l'étape susmentionnée par addition d'un chélatant du zinc, comme par exemple, et de manière non limitative, l'éthylène diamine tétracétate EDTA qui inhibe l'activité métalloprotéasique de la BoNT/A, ou par addition de gangliosides, qui bloquent cette dernière par formation d'un complexe inactif.

19. Kits comprenant un substrat selon l'une des revendications 1 à 12, et, le cas échéant de la BoNT/A, ainsi que les réactifs nécessaires à la détermination des pouvoirs inhibiteurs ou activateurs des molécules testées, ou de la présence de la BoNT/A dans un échantillon déterminé, et, le cas échéant, un fluorimètre, ainsi que les réactifs EDTA et/ou gangliosides séparés ou associés et toutes substances capables d'inhiber les enzymes autres que la BoNT/A sans affecter l'activité de cette dernière.

## Claims

1. Peptide substrate selectively recognized by botulinum toxin type A, BoNT/A, **characterized in that** it comprises in its peptide structure an Nop-(Z)-Pya fragment in which Z represents an amino acids chain, said fragment being cleaved by said toxin.

2. Substrate according to claim 1, **characterized in that** the Nop-(Z)-Pya fragment is chosen from the following:
1) Nop-R-A-Pya (SEQ ID NO: 3)
2) Nop-R-A-T-Pya (SEQ ID NO: 4)
3) Nop-R-A-T-K-Pya (SEQ ID NO: 5)

3. Substrate according to one of claims 1 to 2, **characterized in that** the Nop-(Z)-Pya fragment corresponds to the Nop-R-A-Pya sequence (SEQ ID NO:3).

4. Peptide substrate according to one of claims 1 to 3, selectively recognized by botulinum toxin type A, BoNT/A, **characterized in that** it comprises or is constituted by the following sequence:
Ac-(X)ₘ-S-N-K-T-R-I-D-Xaa-A-N-Nop-R-A-Pya-K-Nle-(L)ₙ(GSG)ₚ-NH₂ (SEQ ID NO: 6)
in which:
- X represents an amino acid, or a chain of 2 to 52 amino acids,
- m, n and p, independently of each other represent 0 or 1,
- Xaa represents E or Q.

5. Substrate according to one of claims 1 to 4, **characterized in that** when m represents 1, X represents:
- an arginine residue or a cysteine residue the thiol function of which is, if appropriate, immobilized on a solid support
- or a chain of 2 to 52 amino acids, corresponding to a peptide fragment of the protein SNAP25 the C-terminal amino acid of which corresponds to the amino acid situated at position 186 of the sequence of the protein SNAP 25, and the N-terminal amino acid of which corresponds to one of the amino acids situated at position 135 to 185 of the sequence of the protein SNAP 25, one or more of the above-mentioned amino acids being replaced, if appropriate, by an amino acid chosen from: A, S, E, F, L, Nle.

6. Substrate according to one of claims 1 to 5, **characterized in that** it comprises or is constituted by the following sequences S 1 to S6:
S1: Ac-S-N-K-T-R-I-D-E-A-N-Nop-R-A-Pya-K-Nle-L-NH₂ (SEQ ID NO: 8)
S2: Ac-C-S-N-K-T-R-I-D-E-A-N-Nop-R-A-Pya-K-Nle-L-NH₂ (SEQ ID NO: 9)
S3: Ac-S-N-K-T-R-I-D-Q-A-N-Nop-R-A-Pya-K-Nle-L-NH₂ (SEQ ID NO: 10)
S4: Ac-C-S-N-K-T-R-I-D-Q-A-N-Nop-R-A-Pya-K-Nle-L-NH₂ (SEQ ID NO: 11)
S5: Ac-S-N-K-T-R-I-D-Q-A-N-Nop-R-A-Pya-K-Nle-NH₂ (SEQ ID NO: 12)
S6: Ac-C-S-N-K-T-R-I-D-Q-A-N-Nop-R-A-Pya-K-Nle-NH₂ (SEQ ID NO: 13)

7. Substrate according to one of claims 1 to 5, **characterized in that** X represents an amino acids chain corresponding to the KAD sequence of SNAP 25 (184-186), or its derived KSD sequence.

8. Substrate according to claim 5, **characterized in that** it comprises or is constituted by the following sequence S7 (SEQ ID NO: 14):
S7: Ac K S D S N K T R I D E A N Nop R A Pya K Nle L G S G NH₂ (SEQ ID NO: 14)

9. Substrate according to one of claims 1 to 5, **characterized in that** X represents an amino acids chain of at least 30 amino acids.

10. Substrate according to claim 9, **characterized in that** X represents an amino acids chains corresponding:
- to the SNAP 25 (135-186) sequence, or its following derived sequences:
- to the SNAP 25 (141-186) sequence, or its following derived sequences:
- to the SNAP 25 (146-186) sequence, or its following derived sequences:
- or to the SNAP 25 (156-186) sequence, or its following derived sequences:
in which Xaa1, Xaa2, Xaa3, and Xaa4, independently of each other, represent M or Nle.

11. Substrate according to claim 9 or 10, **characterized in that** it comprises or is constituted by one of the following sequences S8 to S10:
- S8: SEQ ID NO: 19:
- S9: SEQ ID NO: 20:
- S10: SEQ ID NO: 21:

12. Substrate according to one of claims 9 to 11, **characterized in that** it comprises or is constituted by the following sequence S9 (SEQ ID NO: 20):

13. Non-fluorescent and fluorescent metabolites originating from the cleavage of the peptide substrates defined in one of claims 1 to 12, by BoNT/A, the non-fluorescent metabolites being **characterized in that** they include the Nop residue, and the fluorescent metabolites being **characterized in that** they include the Pya residue.

14. Fluorescent metabolites according to claim 13, originating from the cleavage of the peptide substrates according to any one of claims 1 to 13 by BoNT/A at the level of the bond Nop¹⁹⁷-¹⁹⁸R, i.e. the metabolites of formula:
R-A-Pya-K-Nle-(L)ₙ(GSG)ₚ-NH₂ (SEQ ID NO: 22)
in which n and p are as defined in claim 5.

15. The following fluorescent metabolites according to claim 13 or 14,:
- the following fluorescent metabolite M1, originating from the cleavage of the substrates S1, S2, S3, S4, and S8 to S10 by BoNT/A at the level of the bond Nop¹⁹⁷-¹⁹⁸R,
M1: R-A-Pya-K-Nle-L-NH₂ (SEQ ID NO: 23),
- the following fluorescent metabolite M2, originating from the cleavage of the substrates S5 and S6 by BoNT/A at the level of the bond Nop¹⁹⁷-¹⁹⁸R,
M2: R-A-Pya-K-Nle-NH₂ (SEQ ID NO: 24),
- the following fluorescent metabolite M3, originating from the cleavage of the substrate S7 by BoNT/A at the level of the bond Nop¹⁹⁷-¹⁹⁸R,
M3: R-A-Pya-K-Nle-L-G-S-G-NH₂ (SEQ ID NO: 25).

16. Use of a substrate according to one of claims 1 to 12, for the implementation of methods for the detection, identification and/or assay of BoNT/A or of a compound capable of inhibiting or activating BoNT/A, and for the implementation of *in vitro* methods for diagnosing the presence of BoNT/A in a sample originating from various media such as water, air, milk, drink, food, and biological media such as blood, urine, and cerebrospinal fluid, as well as for the precise determination of BoNT/A concentrations during the production, purification, and conditioning of pharmaceutical compositions for the purposes of therapeutic use in humans or animals, or cosmetic compositions.

17. Method for detecting, identifying and/or assaying a compound capable of inhibiting BoNT/A **characterized in that** it comprises:
- bringing together a substrate according to one of claims 1 to 13 in solution with BoNT/A and at least one compound capable of inhibiting BoNT/A;
- measuring the fluorescence, emitted by the fluorescent metabolites according to one of claims 13 to 15 formed during the preceding stage, in the presence and/or in the absence of said compound, a diminution of fluorescence between the measurement carried out in the presence of this compound and that carried out in the absence of this compound, then indicating that the tested compound is an inhibitor of BoNT/A.

18. *In vitro* method for diagnosing the presence of BoNT/A in a sample as defined in claim 16, or for measuring the concentrations of BoNT/A during the stages of production, purification, and conditioning of pharmaceutical or cosmetic compositions in a sample taken during these stages, comprising:
- a stage of bringing together a substrate according to one of claims 1 to 12 in solution with said sample;
- measurement of the fluorescence emitted by any fluorescent metabolites according to one of claims 13 to 15 formed during the preceding stage, giving evidence of the presence of BoNT/A in said sample, which can be quantified using appropriate standard curves,
- if appropriate and for the purpose of removing falsely positive responses, the elimination of the fluorescence emitted during the above-mentioned stage by the addition of a zinc chelating agent, such as for example, and in non-limiting manner, ethylene diamine tetracetate EDTA which inhibits the metalloprotease activity of BoNT/A, or by the addition of gangliosides, which block the latter by the formation of an inactive complex.

19. Kits comprising a substrate according to one of claims 1 to 12, and, if appropriate, BoNT/A, as well as the reagents necessary for the determination of the inhibiting or activating powers of the tested molecules, or of the presence of BoNT/A in a determined sample, and, if appropriate, a fluorimeter, as well as the reagents EDTA and/or separate or associated gangliosides and any substances capable of inhibiting enzymes other than BoNT/A without affecting the activity of the latter.

## Patentansprüche

1. Peptidsubstrat, das mittels Botulinumtoxin A, BoNT/A, selektiv erkennbar ist, **dadurch gekennzeichnet, dass** es in seiner Peptidstruktur ein Nop-(Z)-Pya-Fragment umfasst, dass durch das Toxin spaltbar ist, und wobei Z für eine Kette aus 2 bis 4 Aminosäuren steht.

2. Substrat nach Anspruch 1, **dadurch gekennzeichnet, dass** Nop-(Z)-Pya aus Folgenden ausgewählt ist:
1) Nop-R-A-Pya (SEQ ID NR.: 3)
2) Nop-R-A-T-Pya (SEQ ID NR.: 4)
3) Nop-R-A-T-K-Pya (SEQ ID NR.: 5).

3. Substrat nach einem der Ansprüche 1 bis 2, **dadurch gekennzeichnet, dass** das Nop-(Z)-Pya-Fragment der Sequenz Nop-R-A-Pya (SEQ ID-NR.: 3) entspricht.

4. Peptidsubstrat nach einem der Ansprüche 1 bis 3, das mittels Botulinumtoxin A, BoNT/A, selektiv erkannt wird, **dadurch gekennzeichnet, dass** es die folgende Sequenz umfasst oder aus ihr besteht: wobei:
- X für eine Aminosäure oder für eine Kette aus 2 bis 52 Aminosäuren steht,
- m, n und p unabhängig voneinander für 0 oder 1 stehen,
- Xaa für E oder Q steht.

5. Substrat nach einem der Ansprüche 1 bis 4, **dadurch gekennzeichnet, dass**, wenn m für 1 steht, X für:
- einen Argininrest oder einen Cysteinrest, deren Thiolfunktion gegebenenfalls auf einem festen Träger immobilisiert ist,
- oder eine Kette aus 2 bis 52 Aminosäuren steht, die einem Peptidfragment des Proteins SNAP25 entsprechen, dessen Aminosäuren-C-Terminus der Aminosäure entspricht, die an Position 186 der Sequenz des Proteins SNAP 25 liegt, und deren Aminosäuren-N-Terminus einer der Aminosäuren entspricht, die an Position 135 bis 185 der Sequenz des Proteins SNAP 25 liegen, wobei eine oder mehrere der oben genannten Aminosäuren gegebenenfalls durch eine Aminosäure ersetzt wird, ausgewählt aus: A, S, E, F, L, Nle.

6. Substrat nach einem der Ansprüche 1 bis 5, **dadurch gekennzeichnet, dass** es die folgenden Sequenzen S1 bis S6 umfasst oder aus ihnen besteht:
S1: Ac-S-N-K-T-R-I-D-E-A-N-Nop-R-A-Pya-K-Nle-L-NH₂ (SEQ ID NR.: 8)
S2: Ac-C-S-N-K-T-R-I-D-E-A-N-Nop-R-A-Pya-K-Nle-L-NH₂ (SEQ ID NR.: 9)
S3: Ac-S-N-K-T-R-I-D-Q-A-N-Nop-R-A-Pya-K-Nle-L-NH₂ (SEQ ID NR.: 10)
S4: Ac-C-S-N-K-T-R-I-D-Q-A-N-Nop-R-A-Pya-K-Nle-L-NH₂ (SEQ ID NR.: 11)
S5: Ac-S-N-K-T-R-I-D-Q-A-N-Nop-R-A-Pya-K-Nle-NH₂ (SEQ ID NR.: 12)
S6: Ac-C-S-N-K-T-R-I-D-Q-A-N-Nop-R-A-Pya-K-Nle-NH₂ (SEQ ID NR.: 13)

7. Substrat nach einem der Ansprüche 1 bis 5, **dadurch gekennzeichnet, dass** X für eine Aminosäurenkette steht, die der Sequenz KAD von SNAP 25 (184 - 186), oder der von KSD abgeleiteten Sequenz entspricht.

8. Substrat nach Anspruch 5, **dadurch gekennzeichnet, dass** es die folgende Sequenz S7 (SEQ ID NR.: 14) umfasst oder aus ihr besteht:

9. Substrat nach einem der Ansprüche 1 bis 5, **dadurch gekennzeichnet, dass** X für eine Kette aus mindestens 30 Aminosäuren steht.

10. Substrat nach Anspruch 9, **dadurch gekennzeichnet, dass** X für eine Aminosäurenkette steht, die:
- der Sequenz SNAP 25 (135-186) oder ihren folgenden abgeleiteten Sequenzen:
- der Sequenz SNAP 25 (141-186) oder ihren folgenden abgeleiteten Sequenzen:
- der Sequenz SNAP 25 (146-186) oder ihren folgenden abgeleiteten Sequenzen:
- oder der Sequenz SNAP 25 (156-186) oder ihren folgenden abgeleiteten Sequenzen:
wobei Xaa1, Xaa2, Xaa3 und Xaa4, unabhängig voneinander, für M oder Nle stehen.

11. Substrat nach den Ansprüchen 9 oder 10, **dadurch gekennzeichnet, dass** es eine der folgenden Sequenzen S8 bis S10 umfasst oder aus ihnen besteht:
- S8: SEQ ID NR.: 19:
- S9: SEQ ID NR.: 20:
- S10: SEQ ID NR.: 21

12. Substrat nach einem der Ansprüche 9 bis 11, **dadurch gekennzeichnet, dass** es die folgende Sequenz S9 (SEQ ID NR.: 20) umfasst oder aus ihr besteht:

13. Fluoreszierende oder nicht fluoreszierende Metaboliten, die aus der Spaltung der Peptidsubstrate, die in einem der Ansprüche 1 bis 12 definiert sind, mittels BoNT/A stammen, wobei die nicht fluoreszierenden Metaboliten **dadurch gekennzeichnet sind, dass** sie den Nop-Rest umfassen, und die fluoreszierenden Metaboliten **dadurch gekennzeichnet sind, dass** sie den Pya-Rest umfassen.

14. Fluoreszierende Metaboliten nach Anspruch 13, die aus der Spaltung der Peptidsubstrate nach irgendeinem der Ansprüche 1 bis 13 durch BoNT/A in Höhe der Nop¹⁹⁷-¹⁹⁸R-Bindung stammen, und zwar, die Metaboliten der Formel:
R-A-Pya-K-Nle-(L)ₙ(GSG)ₚ-NH₂ (SEQ ID NR.: 22)
wobei n und p sind, wie in Anspruch 5 definiert.

15. Folgende fluoreszierende Metaboliten nach Anspruch 13 oder 14:
- folgender fluoreszierende Metabolit M1, der aus der Spaltung der Substrate S1, S2, S3, S4, und S8 bis S10 mittels BoNT/A in Höhe der Nop¹⁹⁷-¹⁹⁸R-Bindung stammt, M1: R-A-Pya-K-Nle-L-NH₂ (SEQ ID NR.: 23),
- folgender fluoreszierende Metabolit M2, der aus der Spaltung der Substrate S5 und S6 mittels BoNT/A in Höhe der Nop¹⁹⁷-¹⁹⁸R-Bindung stammt,
M2: R-A-Pya-K-Nle-NH₂ (SEQ ID NR.: 24),
- folgender fluoreszierende Metabolit M3, der aus der Spaltung des Substrat S7 mittels BoNT/A in Höhe der Nop¹⁹⁷-¹⁹⁸R-Bindung stammt,
M3: R-A-Pya-K-Nle-L-G-S-G-NH₂ (SEQ ID NR.: 25).

16. Verwendung eines Substrats nach einem der Ansprüche 1 bis 12 zur Durchführung von Verfahren zum Nachweis, zur Identifikation und/oder zur Dosierung von BoNT/A oder einer Verbindung, die in der Lage ist, BoNT/A zu hemmen oder zu aktivieren, und zur Durchführung von In-vitro-Methoden zur Diagnose der Gegenwart von BoNT/A in einer Probe, die aus unterschiedlichen Medien, wie etwa Wasser, Luft, Milch, Getränke, Nahrungsmittel und biologischen Medien, wie etwa Blut, Urin und der Gehirn-Rückenmark-Flüssigkeit stammt, sowie zur exakten Bestimmung der BoNT/A-Konzentrationen bei der Fertigung, der Reinigung und der Konditionierung von pharmazeutischen Zusammensetzungen, die der therapeutischen Verwendung bei Mensch oder Tier dienen, oder von kosmetischen Zusammensetzungen.

17. Verfahren zum Nachweisen, Identifizieren und/oder Dosieren einer Verbindung, die in der Lage ist, BoNT/A zu hemmen, **dadurch gekennzeichnet, dass** es umfasst:
- Zusammenbringen in Lösung eines Substrats nach einem der Ansprüche 1 bis 13 mit BoNT/A und mindestens einer Verbindung, die geeignet ist, BoNT/A zu hemmen,
- Messen der Fluoreszenz, die von den fluoreszierenden Metaboliten nach einem der Ansprüche 13 bis 15 emittiert wird, die während des vorhergehenden Schritts gebildet wurden, in Gegenwart und/oder Abwesenheit der genannten Verbindung, Verringerung der Fluoreszenz zwischen der Messung, die in Gegenwart dieser Verbindung ausgeführt wurde und jener, die in Abwesenheit dieser Verbindung ausgeführt wurde, wodurch angezeigt wird, dass die getestete Verbindung ein BoNT/A-Hemmer ist.

18. In-vitro-Methode zur Diagnose der Gegenwart von BoNT/A in einer Probe, wie in Anspruch 16 definiert, oder Messen der BoNT/A-Konzentrationen im Verlauf der Schritte zur Fertigung, Reinigung und Konditionierung von pharmazeutischen oder kosmetischen Zusammensetzungen in einer Probe, die während dieser Schritte entnommen wird, umfassend:
- einen Schritt des Zusammenbringens in Lösung eines Substrats nach einem der Ansprüche 1 bis 12 mit der Probe;
- Messen der Fluoreszenz, die von den möglichen fluoreszierenden Metaboliten nach einem der Ansprüche 13 bis 15 emittiert wird, die während des vorhergehenden Schritts gebildet werden, die die Gegenwart von BoNT/A in der Probe beweisen, die mit Hilfe von geeigneten Eichkurven quantifiziert werden kann,
gegebenenfalls und mit dem Ziel, falsch positive Reaktionen auszuschließen, die Beseitigung der Fluoreszenz, die während des oben genannten Schritts emittiert wird, durch Zugabe eines Chelatbildners für Zink, wie zum Beispiel, aber nicht beschränkt auf Ethylendiamintetraacetat EDTA, das die Metall-Protease-Aktivität von BoNT/A hemmt, oder durch Zugabe von Gangliosiden, die letztgenanntes durch die Bildung eines inaktiven Komplexes blockieren.

19. Kits, umfassend ein Substrat nach einem der Ansprüche 1 bis 12, und gegebenenfalls BoNT/A, sowie die zur Bestimmung der hemmenden oder aktivierenden Kräfte der getesteten Moleküle oder zur Gegenwart von BoNT/A in einer bestimmten Probe notwendigen Reagenzien, und gegebenenfalls ein Fluorimeter, sowie, getrennt oder assoziiert, die Reagenzien EDTA und/oder Ganglioside, und alle Substanzen, die in der Lage sind, Enzyme zu hemmen, die nicht BoNT/A sind, ohne die Aktivität des letztgenannten zu beeinträchtigen.
